(19) Europäisches Patentamt
European Patent Office
Office européen des brevets

(11) **EP 3 503 971 B1**

(12) **EUROPEAN PATENT SPECIFICATION**

(45) Date of publication and mention
of the grant of the patent:
**13.05.2020 Bulletin 2020/20**

(21) Application number: **17720239.7**

(22) Date of filing: **15.03.2017**

(51) Int Cl.:
*A61N 5/10* (2006.01)          *G06T 7/246* (2017.01)
*A61B 6/00* (2006.01)          *G06K 9/62* (2006.01)
*G06T 17/00* (2006.01)

(86) International application number:
**PCT/JP2017/011559**

(87) International publication number:
**WO 2018/037608 (01.03.2018 Gazette 2018/09)**

(54) **MEDICAL IMAGE PROCESSING APPARATUS, TREATMENT SYSTEM, AND MEDICAL IMAGE PROCESSING PROGRAM**

MEDIZINISCHE BILDVERARBEITUNGSVORRICHTUNG, BEHANDLUNGSSYSTEM UND MEDIZINISCHES BILDVERARBEITUNGSPROGRAMM

APPAREIL DE TRAITEMENT D'IMAGE MÉDICALE, SYSTÈME DE TRAITEMENT ET PROGRAMME DE TRAITEMENT D'IMAGE MÉDICALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.08.2016   JP 2016165125**

(43) Date of publication of application:
**03.07.2019 Bulletin 2019/27**

(73) Proprietor: **Kabushiki Kaisha Toshiba Minato-ku
Tokyo 105-8001 (JP)**

(72) Inventors:
• **HIRAI, Ryusuke
Tokyo 105-8001 (JP)**
• **TAGUCHI, Yasunori
Tokyo 105-8001 (JP)**

(74) Representative: **Hoffmann Eitle
Patent- und Rechtsanwälte PartmbB
Arabellastraße 30
81925 München (DE)**

(56) References cited:
WO-A2-2005/032647     US-A1- 2009 005 668
US-A1- 2010 189 218     US-A1- 2012 199 760
US-A1- 2014 371 513     US-A1- 2015 243 025
US-A1- 2016 148 401

**Description**

[Technical Field]

Embodiments of the present invention relate to a medical image processing apparatus, a treatment system, and a medical image processing program.

[Background Art]

Radiotherapy is a treatment method whereby a lesion within the body of a patient is irradiated to destroy the lesion. In radiotherapy, it is necessary to irradiate the position of the lesion accurately. This is because if normal tissue in the body of the patient is irradiated with a radio beam, it can be damaged. For that reason, when radiotherapy is to be performed, computed tomography (CT) is performed beforehand at the treatment planning stage to obtain a three-dimensional grasp of the position of the lesion within the body of the patient. Based on the grasped position of the lesion, the direction and intensity when the radio beam is radiated can be planned so as to reduce the irradiation of normal tissue. At the subsequent treatment stage, the patient is placed so as to match the position of the patient at the treatment planning stage, and the lesion is irradiated with the radio beam, following the direction and intensity of the irradiation established at the treatment planning stage.

In the positioning of the patient at the treatment stage, image matching done between a perspective image of the inside of the patient's body captured with the patient placed on the bed immediately before the start of treatment and a digitally reconstructed radiograph (DRR) image, which is a virtually reconstructed perspective image, from a three-dimensional computer tomography (CT) image captured at the time of treatment planning so as to determine the offset of the position of the patient between these images. By moving the bed based on the determined offset, the position of the lesion and the bones or the like within the body of the patient are adjusted to the positions thereof at the time of treatment planning.

The offset of the patient position is determined by searching for the position in the CT image so that the DRR image most similar to the perspective image is reconstructed. A large number of methods have been proposed for computer automating the search for the patient position. The user (a physician or the like) verifies the result of the automatic search by visually comparing the perspective image with the DRR image prior to the irradiation.

However, there are also cases in which a lesion within the body of a patient is in an organ, such as the lungs or the heart, which moves with the patient's respiration or heartbeat. In such cases, the position of the lesion must be identified even during irradiation with the radio beam. A method for identifying the position of the lesion is to capture a perspective video of the patient even during irradiation by the radio beam and, based on the perspective image, to track the lesion within the body of the patient. There is also a method of indirectly identifying the position of a lesion when the lesion in the patient's body does not appear clearly in the perspective image, by tracking a marker that has been placed in the body of the patient. Methods of irradiating with a radio beam include pursuing irradiation, in which the position of a lesion is tracked and irradiated, and ambush irradiation, in which irradiation is done when the lesion has arrived at the position at the time of treatment planning.

However, there is a possibility of the state of the patient changing suddenly or the patient coughing or sneezing during radio beam irradiation. There are a plurality of other factors causing the failure to track a lesion within the body of the patient. When such an unexpected situation occurs during the radio beam irradiation, it is necessary to immediately stop the irradiation. Given this, abnormality detection that immediately detects when an unexpected situation occurs during radio beam irradiation is required for performing treatment safely.

As an example of an abnormality detection method that detects an unexpected situation occurring during radio beam irradiation, there is the method disclosed in Patent Reference 1. In the method disclosed in Patent Reference 1, the expansion and contraction of the abdomen, the movement of which is linked to the patient's respiration, is monitored, and movement that diverts from the expansion and contraction movement of the abdomen at the time of normal respiration is taken to be abnormal. However, with the method disclosed in Patent Reference 1, the condition within the body of the patient is not monitored. For that reason, with the method disclosed in Patent Reference 1, it is not possible to follow a change in the position of a lesion caused by, for example, the variation in heartbeat or the movement of gas within the bowels of the patient during treatment. Thus, with the method disclosed in Patent Reference 1, there have been cases in which the reliability of abnormality detection has been insufficient.

[Citation List]

[Patent Literature]

[Patent Reference 1] Japanese Patent No. 5925233

Document US2016148401 A1 may be considered to disclose a medical image processing apparatus comprising: a first image acquirer that acquires a plurality of first images of an object to be treated, each of the plurality of first images being captured by a radiographic imaging apparatus at a-respective time of a plurality of times; a second image acquirer that acquires a second image of the object to be treated, the second image being captured by a radiographic imaging apparatus at a time different from the plurality of times when the plurality of first images are captured; a characteristic processor that determines a position of a first characteristic of the object to be treated from the plurality of first images, the characteristic processor being that which determines the position of a second characteristic corresponding to the first characteristic in the object to be treated from the second image; and a determiner that determines whether or not the position of the second characteristic is different, and the determiner being that which outputs a determination signal based on a result of the determination.

[Summary of Invention]

[Solution to Problem]

In some embodiments, a medical image processing apparatus may include, but is not limited to, a first image acquirer that acquires a plurality of first images of an object to be treated, each of the plurality of first images being captured by a radiographic imaging apparatus at a respective time of a plurality of times; a second image acquirer that acquires a second image of the object to be treated ,the second image being captured by a radiographic imaging apparatus at a time different from the plurality of times when the plurality of first images are captured; a characteristic processor that determines a plurality of positions of a first characteristic of the object to be treated from the plurality of first images, the characteristic processor that determines the position of a second characteristic corresponding to the first characteristic in the object to be treated from the second image; a calculator that establishes a first closed space including the plurality of positions of the first characteristic; and a determiner that determines whether or not the position of the second characteristic is within the first closed space, and the determiner that outputs a determination signal, based on a result of the determination.

In some other embodiments, a treatment system may include, but is not limited to, the medical image processing apparatus; a treatment apparatus that has a ray irradiator irradiating the subject to be treated with the treatment beam and the radiographic imaging apparatuses capturing the first and second images; and a controller that controls the irradiation of the treatment beam by the ray irradiator, based on the determination signal.

[Brief Description of the Drawings]

FIG. 1 is a block diagram showing the general constitution of a treatment system that has a medical image processing apparatus of a first, embodiment.

FIG. 2 is a block diagram showing the general constitution of a medical image processing apparatus of the first embodiment.

FIG. 3 is a flowchart showing the flow of state detection processing in a medical image processing apparatus of the first embodiment.

FIG. 4 is a drawing for describing a marker placed in the body of a patient used in processing in a medical image processing apparatus of the first embodiment.

FIG. 5 is a block diagram showing the general constitution of a medical image processing apparatus of a second embodiment.

FIG. 6 is a block diagram showing the general constitution of a medical image processing apparatus of a third embodiment.

FIG. 7 is a block diagram showing the general constitution of a medical image processing apparatus of a fourth embodiment.

FIG. 8 is a block diagram showing the general constitution of a medical image processing apparatus of a fifth embodiment.

[Description of Embodiments]

A medical image processing apparatus, a treatment system, and a medical image processing program of embodiments are described below, with references made to drawings.

(First Embodiment)

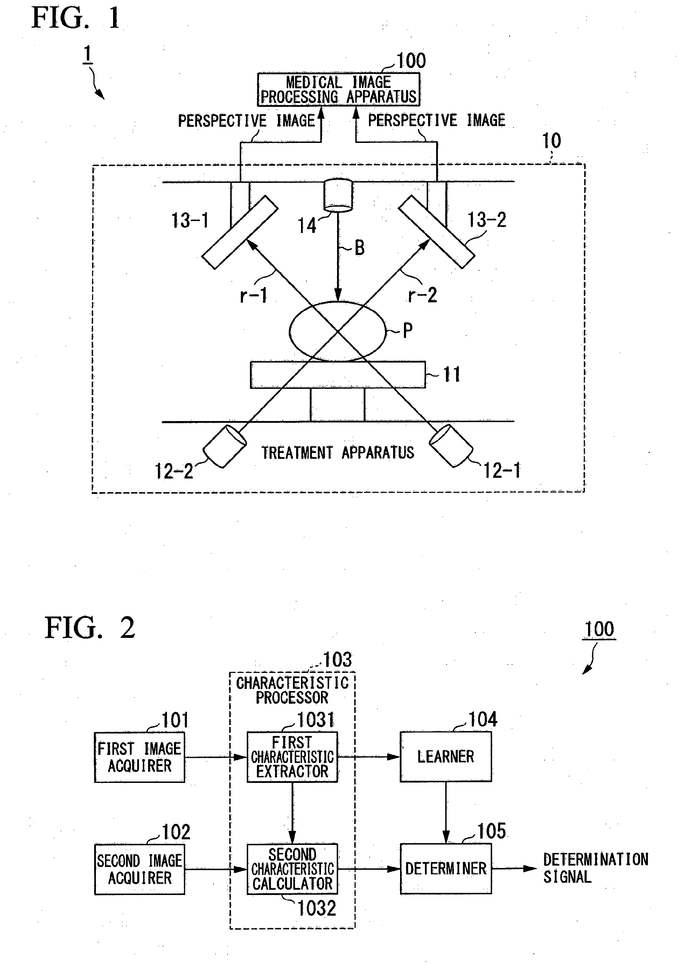

FIG. 1 is a block diagram showing the general constitution of a treatment system that has a medical image processing apparatus of the first embodiment. The treatment system 1 shown in FIG. 1 has a medical image processing apparatus 100 and a treatment apparatus 10.

First, the treatment apparatus 10 of the treatment system 1 will be described. The treatment apparatus 10 has a treatment bed 11, two ray irradiators 12 (ray irradiator 12-1 and ray irradiator 12-2), two ray detectors 13 (ray detector 13-1 and ray detector 13-2), and a treatment beam irradiation gantry 14.

The hyphens (-) and numerals following the reference symbols shown in FIG. 1 are for identifying the relationships of correspondence. For example, in the relationships of correspondence between the ray irradiators 12 and the ray detectors 13 in the treatment apparatus 10, there is correspondence between the ray irradiator 12-1 and the ray detector 13-1, which form one set, and correspondence between the ray irradiator 12-2 and the ray detector 13-2, which form one set. That is, in the description to follow, the hyphen and numerals following each of the reference symbols indicate correspondence between those elements that have the same numeral after the hyphen. In the following description, when referring to a constituent element that exists multiply without distinguishing which one, the hyphen and the numeral following thereafter will be omitted.

The treatment bed 11 is a bed onto which the object to be treated (patient) P by radio beam irradiation is fixed.

The ray irradiator 12-1 irradiates by a radio beam r-1 for viewing inside the body of the patient P from a pre-established angle. The ray irradiator 12-2 irradiates by a radio beam r-2 for viewing inside the body of the patient P from a pre-established angle different from the ray irradiator 12-1. The radio beams r-1 and r-2 are, for example, X-ray beams. In FIG. 1, the case shown is one in which X-ray imaging is performed from two directions with respect to the patient fixed to the treatment bed 11. In FIG. 1, a controller that controls the irradiation by the beams r by the ray irradiators 12 is not shown.

The ray detector 13-1 detects the radio beam r-1 that arrives, having been irradiated from the ray irradiator 12-1 and passed through the body of the patient P, and generates a perspective image within the patient P in accordance with the size of the energy of the detected radio beam r-1. The ray detector 13-2 detects the radio beam r-2 that arrives, having been irradiated from the ray irradiator 12-2 and passed through the body of the patient P, and generates a perspective image within the patient P in accordance with the size of the energy of the detected radio beam r-2. The ray detector 13 has detectors that are arranged in a two-dimensional array and generates as a perspective image a digital image expressed as digital values of the size of the energy of the radio beams r that reach each of the detectors. The ray irradiator 13 is, for example, a flat panel detector (FPD), an image intensifier, or a color image intensifier. The ray irradiator 13 outputs the generated perspective image to the medical image processing apparatus 100.

In FIG. 1, a controller that controls the generation of the perspective image by the ray detector 13 is not shown.

In the treatment apparatus 10 a radiographic imaging apparatus of the treatment system 1 is constituted by one set of the ray irradiator 12 and the ray detector 13.

FIG. 1 shows the constitution of a treatment apparatus 10 having two sets of the ray irradiator 12 and the ray detector 13, that is, two radiographic imaging apparatuses. However, the number of radiographic imaging apparatuses provided in the treatment apparatus 10 is not restricted to the constitution, shown in FIG. 1, which has two radiographic imaging apparatuses, that is, two sets of the ray irradiator 12 and the ray detector 13. For example, the treatment apparatus 10 may have a constitution provided with three or more radiographic imaging apparatuses (three or more sets of the ray irradiator 12 and the ray detector 13). The treatment apparatus 10 may have a constitution having one radiographic imaging apparatus (one set of the ray irradiator 12 and the ray detector 13).

The treatment beam irradiation gantry 14 irradiates by, as treatment beam B, a radio beam for destroying a lesion in the body of the patient P. The treatment beam B, for example, an X-ray beam, a $\gamma$ ray beam, an electron beam, a proton beam, a neutron beam, a heavy particle beam, or the like. In FIG. 1, a controller that controls the irradiation by the treatment beam B by the treatment beam irradiation gantry 14 is not shown.

FIG. 1 shows a constitution of the treatment apparatus 10 that is provided with one treatment beam gantry 14. However, the treatment apparatus 10 is not restricted to a constitution having only one treatment beam irradiation gantry 14 and may have a constitution having a plurality of treatment beam irradiation gantries 14. For example, although FIG. 1 shows the constitution of a treatment apparatus 10 having a treatment beam irradiation gantry 14 that irradiates by the treatment beam B from the vertical direction to the patient P, the treatment system 1 may be constituted to include a treatment apparatus further having a treatment beam irradiation gantry that irradiates by a treatment beam from the horizontal direction to the patient P.

The medical image processing apparatus 100 tracks a lesion in the body of the patient P being treated by radiotherapy, based on the perspective images output from the ray detector 13-1 and the ray detector 13-2. The tracking of the lesion in the medical image processing apparatus 100 is performed based on a perspective image of the patient P captured before performing radiotherapy, such as at the treatment planning stage, and a current perspective image of the patient P.

The medical image processing apparatus 100, based on the results of tracking the lesion in the body of the patient P, automatically detects the timing of irradiating the treatment beam B on the lesion in the radiotherapy. The medical image processing apparatus 100 automatically detects an unexpected situation occurring at the time of tracking a lesion in the body during treatment, based on the results of tracking the lesion in the body of the patient P. The timing of the irradiation by the treatment beam B and the detection of an unexpected situation in the medical image processing apparatus 100 are done by determining a change between the position of the lesion included in a perspective image of the patient P captured before performing radiotherapy and the position of the lesion in the current perspective image of the patient P. The medical image processing apparatus 100 outputs information of the detected timing of irradiation by the treatment beam B and an unexpected situation. An unexpected situation is, for example, the patient P coughing or sneezing, the occurrence of the apnea syndrome when the patient P falls asleep or during sleep, or a sudden change in the condition of the patient P. An unexpected situation includes a system abnormality in the treatment system 1.

The medical image processing apparatus 100 and the ray detector 13 provided in the treatment apparatus 10 may be connected by a LAN (local area network) or a WAN (wide area network).

The constitution of the medical image processing apparatus 100 of the treatment system 1 will now be described. FIG. 2 is a block diagram showing the general constitution of the medical image:processing apparatus 100 of the first embodiment.

The medical image processing apparatus 100 shown in FIG. 2 has a first image acquirer 101, a second image acquirer 102, a characteristic processor 103, a learner 104, and a determiner 105. The characteristic processor 103 has a first characteristic extractor 1031 and a second characteristic calculator 1032.

The first image acquirer 101 acquires a plurality of first images regarding the patient P before treatment. The first image acquirer 101 outputs the plurality of acquired first images to the characteristics processor 103. In this case, a first image is an image based on an image used at the treatment planning stage for performing radiotherapy. At the treatment planning stage, the direction and intensity of the radiation by the treatment beam B is planned to reduce the irradiation by the treatment beam B to normal tissue in the body of the patient P. As one example, the first image may be digitally reconstructed radiograph (DRR) which is prepared from a four-dimensional computed tomography (CT). As one example, the first image is a digitally reconstructed radiograph (DRR) that reconstructs a perspective view virtually from a three-dimensional computed tomography (CT) image captured at the time of treatment planning. The first image may be a perspective image generated by the ray detector 13 in accordance with the radio beam r irradiated by the ray irradiator 12 immediately before the start of treatment after the patient P position is adjusted (state in which the treatment beam irradiation gantry 14 does not irradiate with the treatment beam B). The first image acquirer 101 may include an interface for connecting to the ray detector 13 of the treatment apparatus 10.

The second image acquirer 102 acquires a second image of the patient P captured during treatment. The second image acquirer 102 outputs the captured second image to the characteristic processor 103. In this case, the second image is a perspective image within the body of the patient P captured at each pre-established time interval in the condition in which the patient P is laid down on the treatment bed 11 during treatment. That is, the second image is a perspective image generated by the ray detector 13 in accordance with the radio beam r irradiated by the ray irradiator 12 during treatment (it may be in the condition in which the treatment beam irradiation gantry 14 radiates with the treatment beam B or the condition in which the treatment beam irradiation gantry 14 does not irradiate the treatment beam B). The second image acquirer 102 may have an interface for connection to the ray detector 13 of the treatment apparatus 10.

The characteristics processor 103, based on the plurality of first images output from the first image acquirer 101 and the second image output from the second image acquirer 102, extracts the characteristic subject captured in each image (the first characteristic of the first image and the second characteristic of the second image corresponding to the first characteristic). The characteristic subject indicates a lesion in the body of the patient P, a characteristic location of a body for identifying the position of a lesion in the body of the patient P, or a marker or the like that has been embedded in the body of the patient P, or the like. The characteristic processor 103 outputs information representing the extracted position of lesion to the learner 104 and the determiner 105.

The first characteristic extractor 1031 calculates a characteristic extraction parameter representing a characteristic of a characteristic subject from the first image of the patient P output from the first image acquirer 101, and that outputs the calculated characteristic extraction parameter to the second characteristic calculator 1032. The characteristic subject is, for example, a lesion, a marker, or a part of a subject. The first characteristic extractor 1031, in accordance with the calculated characteristic extraction parameter, extracts the position of the characteristic subject captured in the first image and outputs information representing the extracted characteristic subject position as the first characteristic to the learner 104.

The second characteristic calculator 1032, based on a characteristic extraction parameter output from the first characteristic extractor 1031, extracts the characteristic subject from the second image of the patient P output from the second image acquirer 102. The second characteristic calculator 1032, based on the extracted characteristic subject, calculates the current position of the characteristic subject corresponding to the position of the characteristic subject represented by the first characteristic and outputs information representing the calculated characteristic subject position as the second characteristic to the determiner 105.

The learner (calculator) 104, based on the first characteristic output from the first characteristic extractor 1031 of the characteristic processor 103, learns the change of the position of the characteristic subject at a plurality of times. That is, the learner 104 learns the three-dimensional distribution of the position of the characteristic subject that moves by the movement of the respiration or heartbeat of the patient P. The learner 104, based on the learned distribution of the position of the characteristic subject, establishes a closed space that encompasses the distribution of a plurality of positions of the characteristic subject obtained from the plurality of first images. This closed space represents the expected range of movement of the characteristic subject. The learner 104 outputs information of the established closed space to the determiner 105.

The determiner 105, based on information of the closed space output from the learner 104 and the second characteristic output from the second characteristic calculator 1032 of the characteristic processor 103, determines whether the second characteristic is within the closed space. For example, the determiner 105 determines whether the characteristic subject, within the range of the closed space in which the characteristic subject is expected beforehand to move, has moved to arrive at a position established by irradiation by the treatment beam B at the time of treatment planning. The determiner 105 determines whether the characteristic subject has moved to outside the range of the closed space in which the characteristic subject is expected beforehand to move. The determiner 105 outputs a determination signal representing the result of the determination of the movement of the characteristic subject.

The position irradiated by the treatment beam B established at the time of treatment planning may be a specific position of movement of the characteristic subject, or may be a pre-established range that includes the position of the characteristic subject. In the description to follow, the position and range of the irradiation by the treatment beam B will be referred to as the "gate window." The gate window, as noted above, is the position or range over which the treatment beam B is radiated, which is established at the time of treatment planning. Therefore, the constitution may be such that gate window is set at the time the learner 104 establishes the closed space, and gate window information is output to the determiner 105 together with the closed space information.

The determiner 105, for example, outputs the determination signal to a controller (not shown) that controls the irradiation by the treatment beam B by the treatment beam irradiation gantry 14 of the treatment apparatus 10, and the controller (not shown) that controls the irradiation by the radio beam r by the ray irradiator 12. If radiotherapy is to be done by the treatment system 1, the controller (not shown) performs control so that, when a determination signal is output, indicating that the current position of the characteristic subject moves to within the gate window established in the treatment planning, the treatment beam B is irradiated. If, during radiotherapy in the treatment system 1, a determination signal is output to the controller (not shown) indicating that the current position of the characteristic subject diverts greatly from the gate window established in the treatment planning, the controller (not shown) performs control so as to stop the irradiation by the treatment beam B (which may include the radio beam r).

By this constitution, the medical image processing apparatus 100, based on the first image of the patient P captured before treatment and the second image of the patient P captured during treatment, determines the offset between the gate window established in the treatment planning and the current position of the characteristic subject within the body of the patient P. The medical image processing apparatus 100 outputs as a determination signal the result of determining the offset between the gate window established in the treatment planning and the current position of the characteristic subject. This enables the medical image processing apparatus 100 to automatically detect the timing of irradiation by the treatment beam B of the lesion within the body of the patient P. This enables the treatment system 1 that has the medical image processing apparatus 100 to irradiate the lesion within the body of the patient P using the treatment beam B with the appropriate timing. The medical image processing apparatus 100 can automatically detect unexpected situations during treatment, such as the patient P coughing or sneezing, the occurrence of the apnea syndrome as the patient P falls asleep or during sleep, or a sudden change in the condition of patient P. In the treatment system 1 that has the medical image processing apparatus 100, this enables the development of a strategy for performing treatment safely, such as stopping the irradiation by the treatment beam B when an unexpected situation occurs while irradiating a lesion in the body of the patient P using the treatment beam, and the position of the characteristic subject moves away from the gate window or to the outside of the closed space.

A part of the functional elements of the above-described medical image processing apparatus 100 may be, for example, a software function element that functions by execution of a program stored in storage device by a processor, such as a CPU (central processing unit) or GPU (graphic processing unit). In this case, the storage device may be implemented by a ROM (read-only memory), a RAM (random-access memory), a HD (hard disk) drive, or a flash memory or the like. The program executed by the processor such as a CPU or GPU may be stored beforehand in a storage device of the medical image processing apparatus 100, or may be downloaded from another computer via a network. A program stored in a removable storage device may be installed into the medical image processing apparatus 100. A part or all of the functional elements of the above-noted medical image processing apparatus 100 may be hardware operational units such as an FPGA (field-programmable gate array), an LSI (large-scale integration) device, or an ASIC (application-specific integrated circuit) or the like.

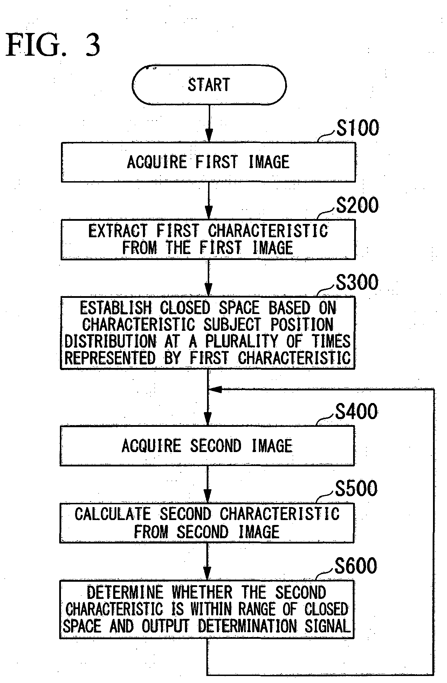

The flow of processing (state detection processing) that detects the state of the characteristic subject in the medical image processing apparatus 100 constituting the treatment system 1 will now be described. The state detection processing in the medical image processing apparatus 100 is processing to determine the current position of the characteristic subject by tracking the characteristic subject. FIG. 3 is a flowchart showing an example of the flow of state detection processing in the medical image processing apparatus 100 of the first embodiment. Before the medical image processing apparatus 100 performs state detection procession, that is, before performing radiotherapy (for example, approximately one week before), a treatment plan is established. Immediately before the medical image processing apparatus 100 performs state detection processing, positioning is done to adjust the position and direction of the patient P when performing treatment with the position and direction of the patient P established in the treatment plan. In the description to follow, it will be assumed that the direction and intensity of the treatment beam B to irradiate the patient P in the treatment plan have already been established, and that the positioning of the patient P to be treated has already been completed. In the positioning of the patient P when performing treatment, the medical image processing apparatus 100 may be used.

First, when the medical image processing apparatus 100 starts the state detection processing, the first image acquirer 101 acquires a first image (step S101). At this point, the first image acquirer 101, to determine whether it is possible to track the characteristic subject properly, first images are acquired for a plurality of respiration periods of the patient P (hereinafter, "respiration period"). As described above, the first image may be a DDR image that is reconstructed perspective image from CT images used at the treatment planning stage, and may be a perspective image captured immediately before the start of treatment, in the state in which the patient P is not being irradiated by the treatment beam B. However, to make a proper determination of the range in which the characteristic subject moves by respiration of the patient P, it is desirable that the first image be images over at least one period of the respiration of the patient P. For that reason, if the first image is a perspective image captured immediately before the start of treatment, the first image acquirer 101 acquires as the first images perspective images (two-direction perspective images) of a plurality of respiration period generated by the ray detector 13 capturing immediately before treatment. That is, the first image acquirer 101 acquires a plurality of two-direction perspective images as the first images. The first image acquirer 101 then outputs each of the acquired first images to the first characteristic extractor 1031 of the characteristic processor 103.

In the radiotherapy, to treat a given patient P, there are cases in which irradiation by the treatment beam B is done a plurality of times (including not on the same day). For that reason, one the second and subsequent radiotherapy with respect to the given patient P, the perspective images (two-direction perspective images) generated by the ray detector 13 by capturing at the time of treatment on the previous time may be used as the first images.

Next, the first characteristic extractor 1031 calculates the characteristic extraction parameters of the characteristic subject from each of the first images output from the first image acquirer 101, and extracts the first characteristic representing the position of the characteristic subject (step S200). In this case, as a method of the first characteristic extractor 1031 extracting the first characteristic from the first images, there is the method, for example, of placing a marker in the body of the patient P as the characteristic subject and extracting the characteristic subject based on the position of the marker. As a method of the first characteristic extractor 1031 extracting the first characteristic from the first images, there is also the method, for example, of taking a part moving in concert with the respiration or heartbeat of the patient P as the characteristic subject and extracting the characteristic subject based on the position of that part. The details regarding the method of the first characteristic extractor 1031 extracting the first characteristic from the first images will be described later. The first characteristic extractor 1031 outputs the calculated characteristic extraction parameter to the second characteristic calculator 1032. The first characteristic extractor 1031 outputs the extracted first characteristic to the learner 104.

Next, the learner 104 learns the position distribution of the characteristic subject at a plurality of times represented by the first characteristic output from the first characteristic extractor 1031 and, based on the learned characteristic subject position distribution, establishes a closed space (step S300). In this case, as a method of the learner 104 learning the position distribution of the characteristic subject, there is, for example, a method of learning the regularity of the position from the distribution of the position of the characteristic subject represented by a plurality of first characteristics and a method of learning a generation model. The details regarding the method of the learner 104 learning the first characteristic and establishing the closed space will be described later. The learner 104 outputs information of the established closed space to the determiner 105.

Next, when the radiotherapy is started, the second image acquirer 102 acquires the second image (step S400). In this case, the second image acquirer 102 acquires perspective images (two-direction perspective image) from the ray detector 13, either at a pre-established interval or consecutively, as the second images for tracking the characteristic subject that moves during treatment. The second images acquired by the second image acquirer 102, except for the timing of capturing, are the same as the perspective images acquired by the first image acquirer 101 from the ray detector 13. The second image acquirer 102 outputs each of the acquired second images to the second characteristic calculator 1032 of the characteristic processor 103.

Next, the second characteristic calculator 1032, based on the characteristic extraction parameter output from the first characteristic extractor 1031, extracts from each of the second images the second characteristic representing the current position of the characteristic subject (step S500). In this case, the method of extracting the second characteristic from the second images by the second characteristic extractor 1032 can be the same as that of the first characteristic extractor 1031. That is, the second characteristic calculator 1032 may, like the first characteristic extractor 1031, for example, extract the second characteristic from the second images by the position of a marker placed in the body of the patient P, or by the position of a part of the body of the patient P that moves in concert with respiration or the heartbeat. The method of the second characteristic calculator 1032 extracting the second characteristic from the second image is not restricted to the same method as that of the first characteristic extractor 1031. That is, if the second characteristic calculator 1032 can extract the second characteristic representing the current position of the characteristic subject from each of the second images, a method different from that of the first characteristic extractor 1031 may be used to extract the second characteristic. In the description to follow, the description is for the case in which the second characteristic calculator 1032 extracts the second characteristic from the second images by a method that is the same as the one by which the first characteristic extractor 1031 extracts the first characteristic from the first images, which will be described later. The second characteristic calculator 1032 outputs the extracted second characteristic to the determiner 105.

Next, the determiner 105 determines whether the second characteristic output from the second characteristic calculator 1032 is within the range of the closed space established by the learner 104, and outputs a determination signal representing the determination result (step S600). More specifically, the determiner 105 outputs a determination signal indicating that an abnormality is not detected if the second characteristic is within the closed space, and outputs a determination signal indicating that an abnormality is detected if the second characteristic is outside the closed space. If a closed space established by a generation model from the learner 104 is output, the determiner 105 calculates a value of probability obtained by inputting to the generation model the second characteristic output from the second characteristic calculator 1032, and determines whether the calculated probability value is within the range of the closed space. The determiner 105 then outputs a determination signal indicating that an abnormality is not detected if the calculated probability value was within the closed space, and outputs a determination signal indicating that an abnormality was detected if the calculated probability value is outside the closed space.

After that, the state detection processing of the medical image processing apparatus 100 returns to step S400, and the processing of step S400 to S600 is repeated. That is, in the state detection processing of the medical image processing apparatus 100, the second image acquirer 102 acquires the next second image and repeats the determination with respect to the second characteristic extracted from that acquired second image.

An example of the processing (processing method) performed by the constituent elements of the medical image processing apparatus 100 in the state detection processing of the medical image processing apparatus 100 of the treatment system 1 will now be described.

First, the method of the first characteristic extractor 1031 extracting the first characteristic from the first images will be described. As described above, as methods of the first characteristic extractor 1031 extracting the first characteristic from the first image, there is a method, for example, of extracting based on the position of the marker placed in the body of the patient P, and the method of extracting based on the position of a part moving in concert with the respiration or heartbeat of the patient P.

The method of extracting the first characteristic based on the position of a marker placed in the body of the patient P will first be described. In this case, a marker is, for example, a spherically shaped or bar-shaped metal object. If a marker is placed in the body of the patient P, the first characteristic extractor 1031 takes the coordinates of the position of the marker captured in each of the first images output from the first image acquirer 101 to be the first characteristic. When this is done, the marker position is taken, for example, as the position of the center of gravity of the marker. The first characteristic extractor 1031, for example, by template matching or the like, identifies the marker position captured in the first image by referencing to a template of the marker image in the perspective image prepared beforehand. The first characteristic extractor 1031 outputs information of the coordinates representing the identified position of the marker as the first characteristic to the learner 104.

The first characteristic extractor 1031 also outputs the coordinates of the identified marker position as the characteristic extraction parameter to the second characteristic calculator 1032. In this case, one characteristic extraction parameter output by the first characteristic extractor 1031 is the image of the region of interest (hereinafter ROI image) for specifying a partial image region that includes the identified marker in one image, that is, in one DRR image selected from a DRR image group of at least one period of the respiration of the patient P. The gate window region (range) determined in the treatment plan is included in the ROI image region. The first characteristic extractor 1031 outputs the ROI images that each correspond to a DRR image of a plurality of DRR images (hereinafter DRR image group) as characteristic extraction parameters to the second characteristic calculator 1032.

If the ROI image group is used as the characteristic extraction parameter, the second characteristic calculator 1032 need only perform processing to extract the second characteristic with respect to only within the range identified in each ROI image. For that reason, in the medical image processing apparatus 100, it is expected that the amount of time required for extraction processing by the second characteristic calculator 1032 to extract the second characteristic from the second images can be shortened.

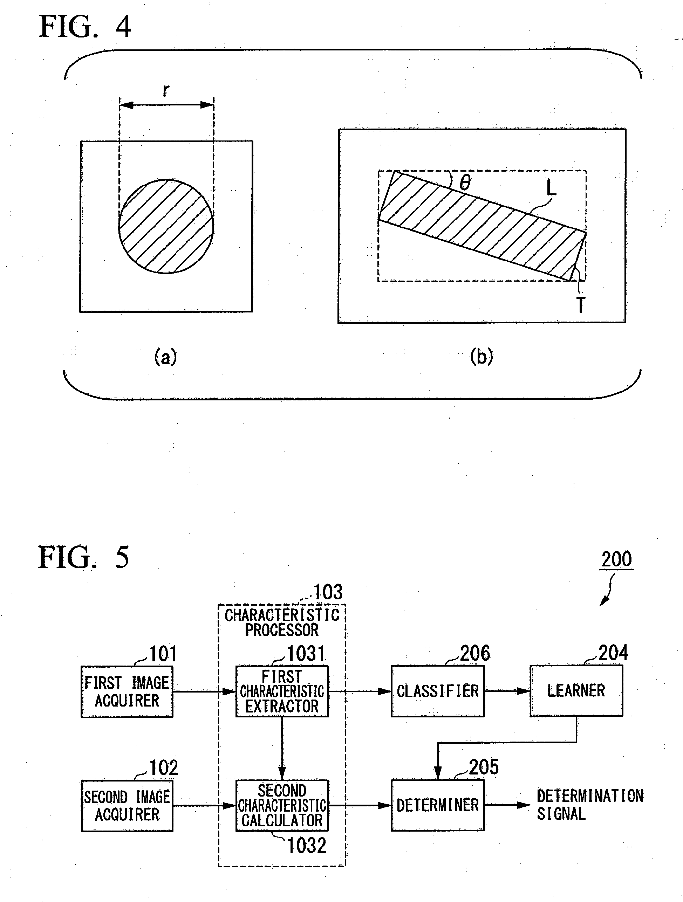

The first characteristic extractor 1031 may output to the second characteristic calculator 1032 a parameter representing the marker image as the characteristic extraction parameter. FIG. 4 describes an example of a marker placed in the body of the patient P that is used in the processing by the medical image processing apparatus 100 of the first embodiment. FIG. 4 shows an example of a marker image captured in a perspective image. In FIG. 4, (a) shows an example of a spherical marker image, and (b) shows an example of a bar-shaped marker image.

If the marked placed in the body of the patient P is a spherical marker, the first characteristic extractor 1031, as shown in FIG. 4 (a), takes the diameter r of the spherical marker to be the characteristic extraction parameter. If the marker place in the body of the patient P is a spherical marker, the characteristic extraction parameter may be the radius of the spherical marker.

If, however, the marker is bar-shaped, the marker image captured in the perspective image changes depending upon the orientation of the marker place in the body of the patient P. For that reason, if the marker placed in the body of the patient P is bar-shaped, the first characteristic extractor 1031, as shown in FIG. 4 (b), takes the angle $\theta$, the length L, and the thickness T of the bar-shaped marker to be the characteristic extraction parameters.

The first characteristic extractor 1031 outputs to the second characteristic calculator 1032 characteristic extraction parameters in accordance with the shape of the marker placed in the body of the patient P. The first characteristic extractor 1031 outputs to the learner 104 the first characteristic extracted in accordance with the characteristic extraction parameters according to the shape of the marker placed in the body of the patient P.

If a plurality of markers are placed in the body of the patient P, the first characteristic extractor 1031 may take characteristics that include the positions of markers captured in the first image as characteristic extraction parameters and extract a plurality of first characteristics in accordance with each of the characteristic extraction parameters.

Next, the method of extracting the first characteristic based on the position of a part that moves in concert with respiration or heartbeat of the patient P will be described. If there is no marker placed in the body of the patient P, the first characteristic extractor 1031, in place of the marker, takes the coordinates of the position of a part that moves in concert with the respiration, heartbeat, or the like in the body of the patient P captured in each of the first images output from the first image acquirer to be the first characteristic. In this case, as a candidate for the part to be used instead of the marker, there is, for example, a boundary of the thoracic diaphragm of the patient P. The first characteristic extractor 1031 outputs to the learner 104 as the first characteristic information of the coordinates representing the position of the part specified in lieu of the marker.

The first characteristic extractor 1031 outputs to the second characteristic calculator 1032 the coordinates of the position of the specified part, as the characteristic extraction parameter. In this case as well, the first characteristic extractor 1031 outputs to the second characteristic calculator 1032 as the characteristic extraction parameter the ROI image group that specifies a partial image region that includes the image of the part specified in each of the plurality of DRR images.

The first characteristic extractor 1031 may take as the characteristic extraction parameter a pixel value at the position of the specified part in the first image, in which case the first characteristic extractor 1031 takes as the first characteristic a vector of one column that has arranged therein the pixel values of the entire fist image or the pixel values within a partial region of the first image. When this is done, the first characteristic extractor 1031 may take as the first characteristic a vector in a first image that has been subjected to processing such as image compression. In this case, the characteristic extraction parameter is expressed as the image region corresponding to the vectorized first image or a parameter in the image compression processing.

The first characteristic extractor 1031 may take as and extract the first characteristic a transformed vector, such as by compressing the dimension of the vector by subjecting a vector set to principal component analysis with respect to pixel values included in the first image. The characteristic extraction parameter in this case is expressed by the axis direction of vector components of each pixel included in the first image.

The first characteristic extractor 1031 may take as the first characteristic a local characteristic quantity determined from the gradient intensity and gradient direction of pixel values used in the field of image recognition. The characteristic extraction parameter in that case is expressed by the method of extracting the local characteristic quantity extracted from the first image.

Next, the method of the learner 104 learning the position distribution of the characteristic subject based on the first characteristic and of establishing a closed space will be described. As described above, as methods for the learner 104 to learn the position distribution of the characteristic subject, there is, for example, a method of learning the regularity of the position from the distribution of the position of the characteristic subject represented by a plurality of first characteristics arid the method of learning a generation model.

First, the method of learning the regularity of the position from the position distribution of the characteristic subject represented by the plurality of first characteristics will be described. If the regularity of the position of the characteristic subject is to be learned, there is a method of the learner 104, for example, in a characteristic space of order n (where n is a natural number and a positive integer) corresponding to the first characteristics, determining an ellipsoid of dimension n (for example three-dimensional) such that encloses all of the plurality of first characteristics output from the first characteristic extractor 1031 on the inside thereof. When this is done, the learner 104 takes the position of the ellipsoid to be the center of gravity of the first characteristic. The learner 104 makes each of the radii that determine the size of the ellipsoid the standard deviation on each axis of the first characteristic. The learner 104 establishes the region of the determined ellipsoid as the closed space. The learner 104 then outputs information of the established closed space to the determiner 105.

The method of learning a generation model from the position distribution of the characteristic subject represented by a plurality of first characteristics will now be described. In the case of learning a generation model, there is a method whereby, the learner 104, taking the generation model to be a multidimensional normal distribution, determines the parameters specifying the normal distribution from the first characteristics, using maximum likelihood estimation. If the generation model is to be learned, the learner 104 may estimate the probability density function of the generation model by kernel density estimation. In these cases, the learner 104, by setting a threshold th such that $0 < th < 1$, establishes the closed space from the generation model. The learner 104 then outputs information of the established closed space to the determiner 105. In the case of learning the generation model, the learner 104, as the information of the established closed space, not only outputs to the determiner 105 the threshold th representing the closed space, but also outputs to the determiner 105 the learned generation model.

The size of the ellipsoid and the ellipsoid region in the case of learning the regularity of the position of the characteristic subject may be established at the treatment planning stage. The value (parameter) of the probability density function in the case of learning a generation model may be established at the treatment planning stage.

As an example of establishing the size of the ellipsoid or ellipsoid region at the treatment planning stage or the parameter of the probability density function, the idea of establishing the radii that determine the size of the ellipsoid region will now be described. In the treatment planning, the energy, irradiation direction, shape of the irradiation range of the treatment beam B (radio beam) to be radiated to the patient P, and distribution of the dose in the case of irradiating by the treatment beam B divided over plurality of times are established. More specifically, first, the proposer of the treatment plan (a physician or the like) specifies, for example, with respect to the CT image captured at the treatment planning stage, the boundary between the region of a tumor (lesion) and normal tissue, and the boundary between the tumor and important organs in the surrounding area. Then, in the treatment planning, based on the depth to the position of the tumor from the surface of the body of the patient P and the size of the tumor, calculated from the information specified regarding the tumor, the intensity, direction, and the like of the treatment beam B to be used in irradiation are determined.

The above-described specification of the boundary between the tumor and normal tissue corresponds to specification of the tumor position and volume. The volume of the tumor is variously referred to as, for example, the gross tumor volume (GTV), the clinical target volume (CTV), the internal target volume (ITV), the planning target volume (PTV). The GTV is the tumor volume that can be verified from an image by the naked eye, which is the volume that requires a sufficient irradiation dose by the treatment beam B in radiotherapy. The CTV is the volume that includes the GTV and the latent tumor to be treated. The ITV is the volume in which a pre-established margin is added to the CTV, giving consideration to the movement of the CTV by the expected physiological movement of the patient P. The PTV is the volume in which a margin is added to the ITV, giving consideration to the error in positioning the patient P when treatment is performed. These volumes are such that they satisfy the following relationship (1).

$$\text{GTV} \in \text{CTV} \in \text{ITV} \in \text{PTV} \qquad \dots (1)$$

For that reason, at the treatment planning stage, each of the radii that determines the size of the ellipsoid may be determined based on a margin that considers the error that possibly occurs in the actual treatment. For example, if the first characteristic is the three-dimensional position of a marker, the sum of the value of the standard deviation and margin of each axis is taken as the radius that determines the size of the ellipsoid. If the first characteristic is extracted from an image of the position captured in the first image, the first characteristic may be extracted with respect to an added image at a position to which parallel translation equal-area deformation is done, based on the size of the margin.

By this kind of processing, in the medical image processing apparatus 100, the learner 104 learns the distribution of the position of the characteristic subject at a plurality of times represented by the first characteristics extracted from the first images and established a closed space. Then, in the medical image processing apparatus 100, the second image acquirer 102 successively acquires the second images, and the determiner 105 determines whether or not the second characteristics extracted from the second images are within the closed space and outputs a determination signal representing the results of the determination. In this manner, in the medical image processing apparatus 100, the position of a lesion within the body of the patient P during treatment is tracked by tracking the characteristic subject. This enables the medical image processing apparatus 100, based on the results of tracking the position of a lesion within the body of the patient P, to automatically detect timing in synchronization with the respiration or heartbeat of the patient P, which is the timing of irradiation of the lesion moving in concert with the respiration or heartbeat of the patient P, by.the treatment beam B. The medical image processing apparatus 100, by tracking the position of a lesion within the body of the patient P, automatically detect unexpected situations occurring the patient P during treatment. In the treatment system 1 that has the medical image processing apparatus 100, this enables irradiation by the treatment beam B when the current position of the lesion is within the gate window detected in the treatment plan, thereby enabling safe radiotherapy.

As described above, in the medical image processing apparatus 100 of the first embodiment, the first image acquirer 101 acquires the first images of the patient P captured before treatment and the first characteristic extractor 1031 of the characteristic processor 103 extracts the first characteristics for identifying the position of the characteristic subject within the body of the patient P. In the medical image processing apparatus 100 of the first embodiment, the learner 104 learns the distribution of the position of the characteristic subject based on the first characteristics, and establishes a closed space representing the range of expected movement of the characteristic subject. In the medical image processing apparatus 100 of the first embodiment, the second image acquirer 102 acquires the second images of the patient P captured during treatment, and the second characteristic calculator 1032 of the characteristic processor 103 calculates the second characteristic representing the current position of the characteristic subject in the body of the patient P. In the medical image processing apparatus 100 of the first embodiment, by the determiner 105 determining whether or not the second characteristic is within the range of the closed space, the position of the characteristic subject within the body of the patient P during treatment is tracked, and a determination signal representing the result of the determination is output. In the treatment system 1 that has the medical image processing apparatus 100 of the first embodiment, based on the results of tracking the position of the characteristic subject within the body of the patient P, this enables the irradiation by the treatment beam B with respect to a lesion that moves in concert with the respiration or heartbeat of a patient P, at an appropriate timing, synchronized to the respiration or heartbeat of the patient P. In the treatment system 1 that has the medical image processing apparatus 100 of the first embodiment, based on the results of tracking the position of the characteristic subject within the body of the patient P, it is possible to detect unexpected situations occurring in the patient P during treatment, such as the occurrence of coughing or sneezing of the patient P, the occurrence of the apnea syndrome as the patient P falls asleep or sleeps, and a sudden change in the condition of the patient P. In the treatment system 1 that has the medical image processing apparatus 100, this enable the development of an appropriate strategy for performing safe radiotherapy, in accordance with the occurrence of unexpected situations detected in the patient P during treatment.

As described above, a medical image processing apparatus 100 has a first image acquirer 101 that acquires a plurality of first images of a patient P captured by a radiographic imaging apparatus at a plurality of times, a second image acquirer 102 that acquires a second image of the patient P captured by a radiographic imaging apparatus at a time different from the first images, a characteristic processor 103 that determines a plurality of positions of a first characteristic of the patient P from the plurality of first images and that determines the position of a second characteristic corresponding to the first characteristic in the body of the patient P from the second image, a calculator (learner 104) that establishes a first closed space including the plurality of positions of the first characteristic, and a determiner 105 that outputs a determination signal, based on the result of determining whether or not the position of the second characteristic is within the first closed space.

As described above, the characteristic processor 103 may have a first characteristic extractor 1031 that calculates a characteristic extraction parameter for extracting a characteristic subject and that outputs information representing the position of the characteristic subject extracted in accordance with the characteristic extraction parameter as the first characteristic, and a second characteristic calculator 1032 that extracts the characteristic subject based on the characteristic extraction parameter and that outputs, as the second characteristic, information representing the position of the characteristic subject corresponding to the first characteristic.

As described above, the determiner 105 may output the determination signal based on the results of determining whether or not the second characteristic is within a second closed space (gate window) included in the first closed space.

As described above, the first characteristic may be a marker in the object to be treated.

As described above, the characteristic extraction parameter may be an image of a region of interest for specifying a partial image region that includes the characteristic subject within the object to be treated captured in the first and second images.

As described above, the treatment system 1 may have a medical image processing apparatus 100, a treatment apparatus 10 that has a ray irradiator (treatment beam irradiation gantry 14) irradiating the patient P with the treatment beam B, and two radiographic imaging apparatuses (two sets of a ray irradiator 12 and a ray detector 13) capturing the first and second images, and a controller 509 that controls the irradiation of the treatment beam B by the treatment beam irradiation gantry 14, based on the determination signal.

The medical image processing apparatus 100 may have a processor such as a CPU or GPU and a storage device such as a ROM, a RAM, a HD drive, or a flash memory, into which is stored a program for causing the processor to function as a first image acquirer 101 that acquires a plurality of first images of a patient P captured by a radiographic imaging apparatus at a plurality of times, a second image acquirer 102 that acquires a second image of the patient P captured by a radiographic imaging apparatus at a time different from the first images, a characteristic processor 103 that determines a plurality of positions of a first characteristic of the patient P from the plurality of first images and that determines the position of a second characteristic corresponding to the first characteristic in the body of the patient P from the second image, a calculator (learner 104) that establishes a first closed space including the plurality of positions of the first characteristic, and a determiner 105 that output a determination signal, based on the result of determining whether or not the position of the second characteristic is within the first closed space.

The medical image processing apparatus 100 may have a processor such as a CPU or GPU and a storage device such as a ROM, a RAM, a HD drive, or a flash memory, into which is stored a program for causing the processor to function as the characteristic processor 103 may have a first characteristic extractor 1031 that calculates a characteristic extraction parameter for extracting the characteristic subject and outputs information representing the position of the characteristic subject extracted in accordance with the characteristic extraction parameter as a first characteristic, and a second characteristic calculator 1032 that extracts the characteristic subject based on the characteristic extraction parameter and outputs information representing the position of the characteristic subject corresponding to the first characteristic as a second characteristic.

(Second Embodiment)

The second embodiment will now be described. The constitution of the treatment system having a medical image processing apparatus of the second embodiment is the constitution of the treatment system 1 having the medical image processing apparatus 100 of the first embodiment shown in FIG. 1, except with the medical image processing 100 replaced by the medical image processing apparatus of the second embodiment (hereinafter the "medical image processing apparatus 200"). In the description to follow, the treatment system having the medical image processing apparatus 200 will be referred to as the treatment system 2.

In the description to follow, of the constituent elements of the treatment system 2 having the medical image processing apparatus 200, constituent elements that are the same as constituent elements of the treatment system 1 having the medical image processing apparatus 100 of the first embodiment are assigned the same reference symbols and the detailed descriptions of those constituent elements will be omitted. In the following, only the constitution, operation, and processing of the medical image processing apparatus 200, which is a constituent element different from the medical image processing apparatus 100 of the first embodiment, will be described.

The medical image processing apparatus 200, similar to the medical image processing apparatus 100 of the first embodiment, tracks the characteristic subject within the body of the patient P treated by radiotherapy, based on a perspective image output from the ray detector 13-1 and the ray detector 13-2. The medical image processing apparatus 200, similar to the medical image processing apparatus 100 of the first embodiment, automatically detects the timing of the irradiation by the treatment beam B and the unexpected situations, based on the results of tracking the characteristic subject within the body of the patient P, and outputs the detected information.

The constitution of the medical image processing apparatus 200 of the treatment system 2 will now be described. FIG. 5 is a block diagram showing the general constitution of the medical image processing apparatus 200 of the second embodiment. The medical image processing apparatus 200 shown in FIG. 5 has a first image acquirer 101, a second image acquirer 102, a characteristic processor 103, a classifier 206, a learner 204, and a determiner 205. The characteristic processor 103 has a first characteristic extractor 1031 and a second characteristic calculator 1032.

The constitution of the medical image processing apparatus 200 is the constitution of the medical image processing apparatus 100 of the first embodiment with the classifier 206 added thereto. Accompanying this, in the medical image processing apparatus 200 has the learner 204 and the determiner 205 in place of the learner 104 and the determiner 105, respectively, of the medical image processing apparatus 100 of the first embodiment. The other constituent elements of the medical image processing apparatus 200 are the same as the constituent elements of the medical image processing apparatus 100 of the first embodiment. In the description to follow, therefore, constituent elements of the medical image processing apparatus 200 that are the same as ones in the medical image processing apparatus 100 of the first embodiment are assigned the same reference symbols, and the detailed descriptions thereof will be omitted. In the description to follow, only the constituent elements that are different from the medical image processing apparatus 100 of the first embodiment will be described.

The characteristic processor 103 outputs to the classifier 206 and the determiner 205 information (first characteristics and second characteristics) representing the position of the characteristic subject extracted based on the first image output from the first image acquirer 101 and the second image output from the second image acquirer 102.

The classifier 206 classifies the first characteristics output from the first characteristic extractor 1031 of the characteristic processor 103 into a plurality of classes, in accordance with the phase (cycle) of the respiration of the patient P, such as the inspiratory and expiratory phases. For example, the classifier 206 uses a data clustering method such as k-means clustering to classify the first characteristic output from the first characteristic extractor 1031 into two classes, which correspond to exhalation and inhalation by the patient P. The classifier 206 outputs to the learner 204 the first characteristic used in the classification and the classification level corresponding to that first characteristic.

In order to represent a state that is intermediate between the exhalation and inhalation by the patient P, the classifier 206, for example, may classify the first characteristic output from the first characteristic extractor 1031 into N classes (where N is a natural number and positive integer). Also, for example, if a respiration sensor or the like is mounted to the patient P, the classifier 206 may use a respiration signal output from the respiration sensor or the like to classify the first characteristic output from the first characteristic extractor 1031 into a plurality of classes. More specifically, the classifier 206 may use the first derivative of a respiration signal output from the respiration sensor at the time the first image is captured that extracts the first characteristic to distinguish between exhalation and inhalation by the patient P and, based on the distinguished result, may classify into two classes, which corresponds to exhalation and inhalation by the patient P.

The learner 204, based on the first characteristic output from the classifier 206, similar to the learner 104 of the medical image processing apparatus 100 of the first embodiment, learns the distribution of the three-dimensional position of the characteristic subject that moves with the movement of the respiration and heartbeat of the patient P at a plurality of times. The learner 204, based on class labels corresponding to the first characteristic output form the classifier 206, classifies the learned characteristic subject position distribution for each class label. The learner 204, based on the distribution of the position of the characteristic subject classified by each class label, establishes a closed space representing the range of movement of the characteristic subject for each class label. The learner 204 outputs to the determiner 205 information of each of the closed spaces established for each class label.

The method of the learner 204 establishing closed spaces corresponding to each of the class labels is the same as that of the learner 104 of the medical image processing apparatus 100 of the first embodiment. If the first characteristics are output from the classifier 206 in time sequence, the learner 204 may determine the change in the time sequence in the class labels to which the first characteristics belong and may output this to the determiner 205 together with the information of each of the closed spaces established for each class label.

The determiner 205, based on the information of the closed spaces of each class label output from the learner 204 and the second characteristic output from the second characteristic calculator 1032 of the characteristic processor 103, similar to the determiner 105 of the medical image processing apparatus 100 of the first embodiment, determines whether or not the second characteristic is within the closed space. However, the determiner 205 makes the determination of whether the second characteristic is within the closed space for each closed space corresponding to each of the class labels. The determiner 205 outputs a determination signal indicating the result of the determination of the characteristic subject movement for each class label.

As described above, the determiner 205 performs a determination of whether the second characteristic is within the closed space for each closed space corresponding to each of the class labels. For that reason, the determiner 205 can, depending upon whether or not the second characteristic is within a closed space corresponding to some class label, determine the state of respiration of the patient P, that is, whether the characteristic subject has moved during treatment, in more detail than the determiner 105 of the medical image processing apparatus 100 of the first embodiment. For example, if the second characteristic output from the second characteristic calculator 1032 is not within a closed space of some class label output from the learner 204, the determiner 205 can output a determination signal indicating that an abnormality has been detected. Also, for example, if it is within a closed space of some class label, the determiner 205 can, based on the class label for which the second characteristic is within the closed space, output a determination signal indicating determination of the respiration state of the patient P. The state of the patient P respiration is, for example, the possibility that the apnea syndrome is occurring in the patient P, or that the respiration of the patient P is irregular.

The method of the determiner 205 determining the second characteristic for determining the respiration state of the patient P will now be described. First, the second characteristic determining method of the case in which the determiner 205 determines the possibility of the apnea syndrome of the patient P occurring will be described. In this case, the determiner 205 determines whether or not the second characteristic is within a closed space corresponding to some class label, and temporarily stores the information of the class label for which the determination is made that the second characteristic is within the class label. If the determination is made that the second characteristic is within the closed spaces of a plurality of class labels, the determiner 205 temporarily stores the information of all the class labels for which the determination is made that the second characteristic is within the closed space. After that, the determiner 205 determines whether or not, within a pre-established period of time, another second characteristic is within a closed space corresponding to some class label. As a result, if a determination is made that another second characteristic is within a closed space of the same class label as the temporarily stored class label, the determiner 205 determines that the patient P is not breathing periodically, that is, that the patient P might be experiencing the apnea syndrome, and outputs a determination signal indicating that an abnormality has been detected. If, however, a determination is made that another second characteristic is within a closed space of the different class label from the temporarily stored class label, the determiner 205 outputs a determination signal indicating that an abnormality has not been detected. In this manner, the determiner 205 can, based on information of a class label for which the determination is made that a plurality of second characteristics are within a closed space within a pre-established period of time, output a determination signal that includes determination of the possibility that the patient P is experiencing the apnea syndrome.

Continuing, the method of the determiner 205 determining the second characteristic in the case of determining the possibility of irregular respiration of the patient P will be described. In this case, the determiner 205 determines whether or not the second characteristic is within a closed space corresponding to some class label, and temporarily stores the change of the time sequence of class label for which the determination is made that a second characteristic is within a closed space. Then, if the change of the time sequence of temporarily stored class labels is different from the change of the time sequence of the class labels to which the first characteristics output by the learner 204 belong, the determiner 205 determines that there is a possibility that the respiration period of the patient P is irregular, and outputs a determination signal indicating that an abnormality has been detected. If, however, the change of the time sequence of the temporarily stored class label is the same as the change of the time sequence of the class labels to which the first characteristics output by the learner 204 belong, the determiner 205 outputs a determination signal indicating that an abnormality has not been detected. In this manner, the determiner 205, using information output from the learner 204 regarding the change of the time sequence in the class labels to which each first characteristic belongs, can output a determination signal that includes determination of the possibility of the respiration of the patient P being irregular.

By this constitution and operation, the medical image processing apparatus 200, based on the first image of the patient P captured before treatment and the second image of the patient P captured during treatment, outputs as a determination signal the result of determining, for each class label, the offset between the gate window established in the treatment plan and the current position of the characteristic subject in the body of the patient P. By doing this, in the treatment system 2 that has the medical image processing apparatus 200, similar to the treatment system 1 that has the medical image processing apparatus 100 of the first embodiment, it is possible to irradiate with the treatment beam B at an appropriate timing synchronized to the respiration and heartbeat of the patient P and to develop a strategy for a case in which an unexpected situation occurred in a patient P during treatment.

Furthermore, in the medical image processing apparatus 200, the classifier 206 classifies the first characteristics into a plurality of classes in accordance with the phase (period) of respiration such as the exhalation and inhalation of the patient P, and the learner 204 and determiner 205 each perform processing based on the first characteristics classified into a plurality of classes. By doing this, with the medical image processing apparatus 200, it is possible to track the position of a characteristic subject in the body of a patient P during treatment, including whether or not a respiration state of the patient P is occurring periodically. Stated differently, with the medical image processing apparatus 200, it is possible to detect with high accuracy an unexpected situation occurring in the patient P during treatment and timing of irradiation of a lesion within the body of the patient P by the treatment beam B. This enables the treatment system 2 that has the medical image processing apparatus 200 to perform radiotherapy safely.

The state detection processing in the medical image processing apparatus 200 can be easily thought of as the state detection processing in the medical image processing apparatus 100 of the first embodiment shown in FIG. 3, to which is added processing by the classifier 206. More specifically, it is easy to think of this as the processing by the classifier 206 being added between step S200 and step S300 in the state detection processing of the medical image processing apparatus 100 of the first embodiment shown in FIG. 3. Therefore, the detailed description regarding the flow of state detection processing in the medical image processing apparatus 200 will be omitted.

As described above, in the medical image processing apparatus 200 of the second embodiment, the first image acquirer 101 acquires the first image of the patient P captured before treatment, and the first characteristic extractor 1031 of the characteristic processor 103 extracts the first characteristics for identifying the position of the characteristic subject in the body of the patient P. In the medical image processing apparatus 200 of the second embodiment, the classifier 206 classifies the first characteristics into a plurality of classes, in accordance with the phase (period) of respiration, such as exhalation and inhalation of the patient P. In the medical image processing apparatus 200 of the second embodiment, the learner 204 learns the distribution of the position of the characteristic subject based on the classified first characteristics, and establishes a closed space representing the expected range of movement of the characteristic subject for each class label. In the medical image processing apparatus 200 of the second embodiment, the second image acquirer 102 acquires the second image of the patient P captured during treatment, and the second characteristic calculator 1032 of the characteristic processor 103 calculates the second characteristic representing the current position of the characteristic subject in the body of the patient P. In the medical image processing apparatus 200 of the second embodiment, the determiner 205, by determining whether or not the second characteristic is within the closed space for each class label, tracks the position of the characteristic subject within the body of the patient P during treatment, and outputs a determination signal representing the result of that determination. By doing this, with the treatment system 2 that has the medical image processing apparatus 200 of the second embodiment, similar to the treatment system 1 that has the medical image processing apparatus 100 of the first embodiment, it is possible to irradiate with the treatment beam B with an appropriate timing synchronized to the exhalation and inhalation of the patient P. With the medical image processing apparatus 200 of the second embodiment, by performing tracking of the position of the characteristic subject within the body of the patient P for each class label, it is possible to detect an unexpected situation occurring in the patient P, including the state of respiration of the patient P during treatment. With the treatment system 2 that has the medical image processing apparatus 200 of the second embodiment, a proper strategy can be developed for performing safe radiotherapy, in accordance with a detected unexpected situation occurring in a patient P during treatment.

In the medical image processing apparatus 200 of the second embodiment, with the constitution of the medical image processing apparatus 200 shown in FIG. 5, the description has been for the classifier 206 being a separate constituent element of the medical image processing apparatus 200. The classifier 206, however, is not restricted to being a separate constituent element in the medical image processing apparatus 200. For example, in the medical image processing apparatus 200, the function of the classifier 206 may be constituted as a function of the learner 204.

As described above, the medical image processing apparatus 200 further has the classifier 206 that classifies the plurality of first characteristic positions into two or more classes, thereby setting the second closed space from the first closed space.

(Third Embodiment)

The third embodiment will now be described. The constitution of a treatment system having a medical image processing apparatus of the third embodiment is the constitution of the treatment system 1 having the medical image processing apparatus 100 of the first embodiment shown in FIG. 1, with the medical image processing apparatus 100 replaced by the medical image processing apparatus of the third embodiment (hereinafter, the "medical image processing apparatus 300"). In the description to follow, the treatment system having the medical image processing apparatus 300 will be referred to as the treatment system 3.

In the description to follow, of the constituent elements of the treatment system 3 having the medical image processing apparatus 300, constituent elements that are the same as constituent elements of the treatment system 1 having the medical image processing apparatus 100 of the first embodiment are assigned the same reference symbols and the detailed descriptions of those constituent elements will be omitted. In the following, only the constitution, operation, and processing of the medical image processing apparatus 300, which is a constituent element different from the medical image processing apparatus 100 of the first embodiment, will be described.

The medical image processing apparatus 300, similar to the medical image processing apparatus 100 of the first embodiment, tracks the characteristic subject within the body of the patient P treated by radiotherapy, based on a perspective image output from the ray detector 13-1 and the ray detector 13-2. The medical image processing apparatus 300, similar to the medical image processing apparatus 100 of the first embodiment, automatically detects the timing of the irradiation with the treatment beam B and the unexpected situations, based on the results of tracking the characteristic subject within the body of the patient P, and outputs the detected information.

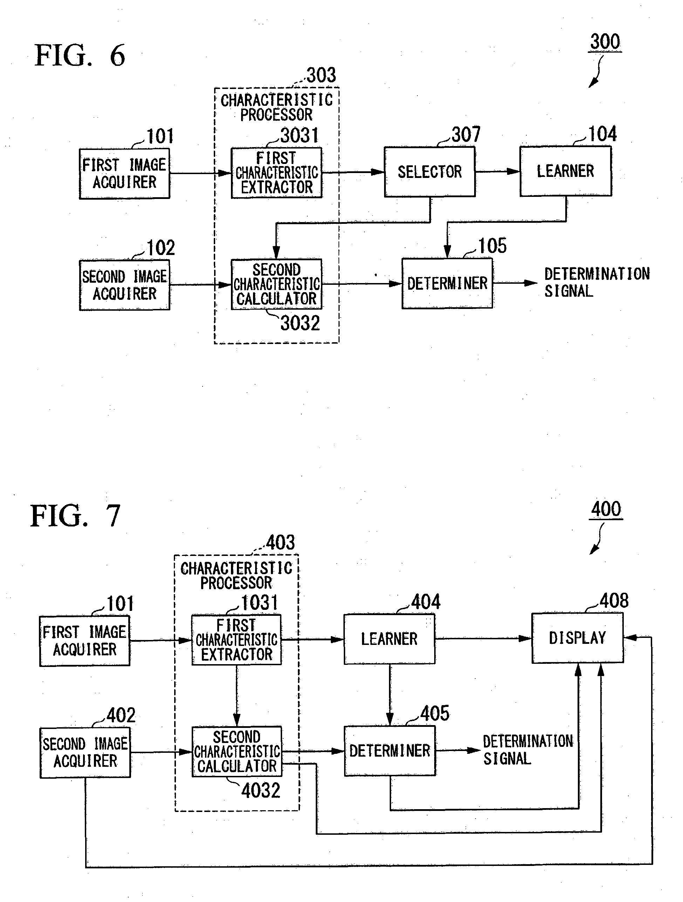

The constitution of the medical image processing apparatus 300 of the treatment system 3 will now be described. FIG. 6 is a block diagram showing the general constitution of the medical image processing apparatus 300 of the third embodiment. The medical image processing apparatus 300 shown in FIG. 6 has a first image acquirer 101, a second image acquirer 102, a characteristic processor 303, a selector 307, a learner 104, and a determiner 105. The characteristic processor 303 has a first characteristic extractor 3031 and a second characteristic calculator 3032.

The constitution of the medical image processing apparatus 300 is the constitution of the medical image processing apparatus 100 of the first embodiment with the selector 307 added thereto. Accompanying this, the medical image processing apparatus 300 has the characteristic processor 303 in place of the characteristic processor 103 of the medical image processing apparatus 100 of the first embodiment. The characteristic processor 303 also has the first characteristic extractor 3031 and the second characteristic calculator 3032 in place of the first characteristic extractor 1031 and the second characteristic calculator 1032 of the characteristic processor 103. The other constituent elements of the medical image processing apparatus 300 are the same as the constituent elements of the medical image processing apparatus 100 of the first embodiment. In the description to follow, therefore, constituent elements of the medical image processing apparatus 300 that are the same as ones in the medical image processing apparatus 100 of the first embodiment are assigned the same reference symbols, and the detailed descriptions thereof will be omitted. In the description to follow, only the constituent elements that are different from the medical image processing apparatus 100 of the first embodiment will be described.

The first image acquirer 101 acquires first images of the patient P captured before treatment and outputs them to the characteristic processor 303.

The second image acquirer 102 acquires the second images of the patient P captured during treatment and outputs them to the characteristic processor 303.

The characteristic processor 303, similar to the characteristic processor 103 of the medical image processing apparatus 100 of the first embodiment, based on the first images output from the first image acquirer 101 and the second images outputs from the second image acquirer 102, extracts the characteristic for identifying the position of the characteristic subject within the body of the patient P. The characteristic processor 303 outputs to the selector 307 and the determiner 105 information (the first and second characteristics) that represents the position of extracted characteristic subject.

The first characteristic extractor 3031, similar to the first characteristic extractor 1031 within the characteristic processor 103 of the medical image processing apparatus 100 of the first embodiment, calculates the characteristic extraction parameters representing the characteristics of the characteristic subject from the first images of the patient P. When this is done, the first characteristic extractor 3031 calculates a plurality (two or more) characteristic extraction parameters. The first characteristic extractor 3031 outputs each of the calculated characteristic extraction parameters to the selector 307. The first characteristic extractor 3031, in accordance with each of the calculated characteristic extraction parameters, extracts the position of the characteristic subject captured in the first images and outputs each of the first characteristics that represent the extracted position of the characteristic subject to the selector 307. The method of the first characteristic extractor 3031 extracting the first characteristic from the first images in accordance with the characteristic extraction parameters is the same as the first characteristic extractor 1031 in the characteristic processor 103 of the medical image processing apparatus 100 of the first embodiment.

The second characteristic calculator 3032 extracts the characteristic subject from the second image of the patient P, based on a characteristic extraction parameter output from the selector 307, that is, based on one of the characteristic extraction parameters output from the first characteristic extractor 3031. The second characteristic calculator 3032 calculates the position of extracted characteristic subject and outputs information indicating the calculated characteristic subject position to the determiner 105 as the second characteristic. The method of the second characteristic calculator 3032 extracting the second characteristic from the second image based on the characteristic extraction parameters is the same as that of the second characteristic calculator 1032 in the characteristic processor 103 of the medical image processing apparatus 100 of the first embodiment.

The selector 307 acquires a plurality of characteristic extraction parameter output from the first characteristic extractor 3031 of the characteristic processor 303 and the first characteristics. The selector 307, based on the distribution of the position of the characteristic subject at a plurality of times represented by each of the acquired first characteristics, evaluates each of the corresponding characteristic extraction parameters and, based on that evaluation, selects the characteristic extraction parameter preferable for extraction of the characteristic subject. The selector 307 outputs the selected characteristic extraction parameter to the second characteristic calculator 3032 of the characteristic processor 303. The selector 307 selects a first characteristic corresponding to the characteristic extraction parameter output to the second characteristic calculator 3032, that is a preferable first characteristic extracted by the preferable characteristic extraction parameter, and outputs the first characteristic to the learner 104.

The method of the selector 307 evaluating the characteristic extraction parameters based on the first characteristics will now be described. The first characteristics are information representing the position of the characteristic subject extracted from a first image captured before radiotherapy, in the state in which the patient P respiration has a stable period. For that reason, it is desirable that the distribution of the position of the characteristic subject at a plurality of times represented by the first characteristic have a high density. That is, it is desirable that the variance value for each of the-components in the first characteristics be as small as possible. Given this, the selector 307, for each characteristic extraction parameter, determines the variable of components representing the position of the characteristic subject in the first characteristics at a plurality of times. When this is done, because the characteristic space to which the first characteristics belong differs for each of the characteristic extraction parameters that extracted the first characteristics, it is not possible to simply compare the relative sizes of the components that represent the positions of the characteristic subject in the first characteristics. For that reason, the selector 307, for each characteristic extraction parameter, calculates maximum variance value $\sigma1$ representing the position of the characteristic subject in the corresponding first characteristic and the next largest variance value $\sigma2$ and compares the ratio between the variance value $\sigma1$ and variance value $\sigma2$ ($\sigma2/\sigma1$). Then, the selector 307 selects and outputs to the second characteristic calculator 3032 the characteristic extraction parameter corresponding to the first characteristic having the largest variance value ratio of the compared variance values as the preferable characteristic extraction parameter. The selector 307 outputs to the learner 104 the first characteristic corresponding to the characteristic extraction parameter selected as the preferable characteristic extraction parameter.

By doing this, the second characteristic calculator 3032, based on the preferable characteristic extraction parameter output from the selector 307, outputs to the determiner 105 the second characteristic, which represents the position of the characteristic subject extracted from the second characteristic of the patient P. The learner 104 outputs to the determiner 105 information of the closed space established using the preferable first characteristic extracted by the preferable characteristic extraction parameter.

By this constitution and operation, the medical image processing apparatus 300, similar to the medical image processing apparatus 100 of the first embodiment, based on the first image of the patient P captured before treatment and the second image of the patient P captured during treatment, outputs as a determination signal the result of determining the offset between the gate window established in the treatment plan and the current position of the characteristic subject in the body of the patient P. By doing this, in the treatment system 3 that has the medical image processing apparatus 300, similar to the treatment system 1 that has the medical image processing apparatus 100, it is possible to perform irradiation at an appropriate timing synchronized to the exhalation and inhalation by the patient P and to develop a strategy for a case in which an unexpected situation occurring in a patient P is detected during treatment.

Furthermore, in the medical image processing apparatus 300, the selector 307 evaluates a plurality of characteristic extraction parameters and selects a characteristic extraction parameter preferable for extraction of the characteristic subject, and the learner 104, based on the first characteristic extracted by the preferable characteristic extraction parameter, establishes a closed space. By doing this, with the medical image processing apparatus 300, it is possible to track the position of a characteristic subject in the body of a patient P during treatment with higher accuracy than the medical image processing apparatus 100 of the first embodiment. Stated differently, with the medical image processing apparatus 300, it is possible to detect with high accuracy the timing of irradiation of a lesion within the body of the patient P by the treatment beam B and an unexpected situation occurring in the patient P during treatment. This enables the treatment system 3 that has the medical image processing apparatus 300 to perform radiotherapy safely.

The state detection processing in the medical image processing apparatus 300 can be thought of the same as the state detection processing in the medical image processing apparatus 100 of the first embodiment. More specifically, it is easy to think of this as the processing by the selector 307 being included in the processing of step S500 and step S300 in the state detection processing of the medical image processing apparatus 100 of the first embodiment shown in FIG. 3. Therefore, the detailed description regarding the flow of state detection processing in the medical image processing apparatus 300 will be omitted.

As described above, in the medical image processing apparatus 300 of the third embodiment, the first image acquirer 101 acquires the first image of the patient P captured before treatment, and the first characteristic extractor 3031 and the selector 307 of the characteristic processor 303 extract a preferable first characteristic extraction parameter for identifying the position of the characteristic subject within the body of the patient P. In the medical image processing apparatus 300 of the third embodiment, the learner 104 learns the distribution of the position of the characteristic subject based on the preferable first characteristic, and establishes a closed space that represents the expected range of movement of the characteristic subject. In the medical image processing apparatus 300 of the third embodiment, the second image acquirer 102 acquires a second image of the patient P captured during treatment, and the second characteristic calculator 3032 of the characteristic processor 303, based on the preferable characteristic extraction parameter, calculates the second characteristic that represents the current position of the characteristic subject within the body of the patient P. In the medical image processing apparatus 300 of the third embodiment, by determining whether or not the second characteristic is within range of the closed space, the determiner 105 tracks the position of the characteristic subject within the body of the patient P during treatment with greater accuracy than the medical image processing apparatus 100 of the first embodiment, and outputs a determination signal that represents the determination result. In the treatment system 3 that has the medical image processing apparatus 300 of the third embodiment, this enables irradiation by the treatment beam B of a lesion that moves in concert with the respiration and heartbeat of the patient P, with an appropriate timing synchronized to the exhalation and inhalation by the patient P and an accuracy higher than that of the treatment system 1 that has the medical image processing apparatus 100 of the first embodiment. With the medical image processing apparatus 300 of the third embodiment, based on the results of tracking the position of the characteristic subject within the body of the patient P with high accuracy, it is possible to detect an unexpected situation occurring in the patient P during treatment. In the treatment system 3 that has the medical image processing apparatus 300 of the third embodiment, this enables the development of an appropriate strategy for performing radiotherapy safely in accordance with an unexpected situation occurring in a patient P detected with high accuracy.

In the medical image-processing apparatus 300 of the third embodiment, with the constitution of the medical image processing apparatus 300 shown in FIG. 6, the description has been for the selector 307 being a separate constituent element of the medical image processing apparatus 300. The selector 307, however, is not restricted to being a separate constituent element of the medical image processing apparatus 300. For example, in the medical image processing apparatus 300, the function of the selector 307 may be constituted as a function of the first characteristic extractor 3031.

As described above, in the medical image processing apparatus 300, the first characteristic extractor 3031 calculates a plurality of characteristic extraction parameters, and outputs a plurality of first characteristics corresponding to each of the characteristic extraction parameters, and the medical image processing apparatus 300 further has a selector 307 that, based on the distribution of the position of the characteristic subject represented by each of the plurality of first characteristics, selects characteristic extraction parameters used by the second characteristic calculator 3032 to extract the characteristic subject and the first characteristic used by a calculator (learner 104) to establish a closed space.

(Fourth Embodiment)

The fourth embodiment will now be described. The constitution of a treatment system having a medical image processing apparatus of the fourth embodiment is the constitution of the treatment system 1 having the medical image processing apparatus 100 of the first embodiment shown in FIG. 1, with the medical image processing apparatus 100 replaced by the medical image processing apparatus of the fourth embodiment (hereinafter, the "medical image processing apparatus 400"). In the description to follow, the treatment system having the medical image processing apparatus 400 will be referred to as the treatment system 4.

In the description to follow, of the constituent elements of the treatment system 4 having the medical image processing apparatus 400, constituent elements that are the same as constituent elements of the treatment system 1 having the medical image processing apparatus 100 of the first embodiment are assigned the same reference symbols and the detailed descriptions of those constituent elements will be omitted. In the following, only the constitution, operation, and processing of the medical image processing apparatus 400, which is a constituent element different from the medical image processing apparatus 100 of the first embodiment, will be described.

The medical image processing apparatus 400, similar to the medical image processing apparatus 100 of the first embodiment, tracks the characteristic subject within the body of the patient P to be treated by radiotherapy, automatically detects the timing of the irradiation by the treatment beam B and an unexpected situation, and outputs the detected information. The medical image processing apparatus 400, by displaying the state of the tracking of the characteristic subject within the body of the patient P, presents it to a user (a physician or the like) of the treatment system 4 that has the medical image processing apparatus 400.

The constitution of the medical image processing apparatus 400 of the treatment system 4 will now be described. FIG. 7 is a block diagram showing the general constitution of the medical image processing apparatus 400 of the fourth embodiment. The medical image processing apparatus 400 shown in FIG. 7 has a first image acquirer 101, a second image acquirer 402, a characteristic processor 403, a learner 404, a determiner 405, and a display 408. The characteristic,processor 403 has, a first characteristic extractor 1031 and a second characteristic calculator 4032.

The medical image processing apparatus 400 has a constitution of the medical image processing apparatus 100 of the first embodiment with the display 408 added thereto. Accompanying this, the characteristic processor 103, the learner 104, and the determiner 105 of the medical image processing apparatus 100 of the first embodiment are replaced by the characteristic processor 403, the learner 404, and determiner 405, respectively. In the characteristic processor 403, the second characteristic calculator 1032 of the characteristic processor 103 is replaced by the second characteristic calculator 4032. The other constituent elements of the medical image processing apparatus 400 are the same as the constituent elements of the medical image processing apparatus 100 of the first embodiment. Therefore, in the description to follow, the constituent elements of the medical image processing apparatus 400 that are the same as ones of the medical image processing apparatus 100 of the first embodiment are assigned the same reference symbols, and the detailed descriptions thereof will be omitted. In the following description, only constituent elements that are different from those of the medical image processing apparatus 100 of the first embodiment will be described.

The first image acquirer 101 acquires and outputs to the characteristic processor 403 a first image of a patient P captured before treatment.

The second image acquirer 402 acquires and outputs to the characteristic processor 403 a second image of the patient P captured during treatment. The second image acquirer 402 outputs the captured second image to the display 408. In this case, the second image acquirer 402 outputs to the display 408 as the second images two perspective images (two-direction perspective images) generated by the ray irradiator 13-1 and the ray irradiator 13-2, respectively.

The characteristic processor 403, similar to the characteristic processor 103 of the medical image processing apparatus 100 of the first embodiment, outputs to the learner 404 and the determiner 405 information indicating the position of the characteristic subject (first and second characteristics), which has been extracted based on the first image output from the first image acquirer 101 and the second image output from the second image acquirer 402. The characteristic processor 403 outputs the second characteristic to the display 408.

The first characteristic extractor 1031 outputs a characteristic extraction parameter representing the characteristic of the characteristic subject from the first image of the patient P to the second characteristic calculator 4032. The first characteristic extractor 1031, outputs to the learner 404 a first characteristic that extracted, in accordance with the characteristic extraction parameter, the position of the characteristic subject captured in the first image.

The second characteristic calculator 4032, similar to the second characteristic calculator 1032 in the characteristic processor 103 of the medical image processing apparatus 100 of the first embodiment, outputs to the determiner 405 a second image representing the position of the characteristic subject extracted from the second image based on the characteristic extraction parameter output from the first characteristic extractor 1031. The second characteristic calculator 4032 outputs the second characteristic to the display 408.

The learner 404, based on the first characteristic output from the first characteristic extractor 1031 of the characteristic processor 403, learns the distribution of the three-dimensional position of the characteristic subject that moves with the respiration and heartbeat of the patient P at a plurality of times and establishes a closed space. The learner 404 outputs information of the established closed space to the determiner 405. The learner 404 may, in addition to the information of the closed space, output information of the gate window to the determiner 405. The methods of the learner 404 learning the change of position of the characteristic subject at the plurality of times based on the first image and establishing the closed space based on the distribution of the position of the characteristic subject learned by the learner 404 are the same as the method of the learner 104 of the medical image processing apparatus 100 of the first embodiment. The learner 404 outputs information of the established closed space to the display 408. The learner 404 may, in addition to the information of the closed space, output information of the gate window to the display 408.

The determiner 405, based on information of the closed space output from the learner 404 and the second characteristic output from the second characteristic calculator 4032 of the characteristic processor 403, determines whether or not the second characteristic is within the closed space and outputs a determination signal indicating the determined results. The method of the determiner 405 determining whether or not the second characteristic is within the closed space is the same as the method of the determiner 105 of the medical image processing apparatus 100 of the first embodiment. The determiner 405 outputs to the display 408 information for presenting the results of determining whether or not the second characteristic is within the closed space, that is, information represented by the determination signal. The determiner 405 may calculate the distance between the position of the characteristic subject represented by the second characteristic and the boundary of the range of movement of the characteristic subject represented by the closed space and output information of the calculated distance, together with the information represented by the determination signal to the display 408.

The display 408 is a display user interface having a display device such as an LCD (liquid crystal display). The display 408 displays an image for the purpose of presenting each of the second image output from the second image acquirer 402, the second characteristic output from the second characteristic calculator 4032, information of the closed space output from the learner 404, and information represented by the determination signal output from the determiner 405.

The method of the display 408 displaying each of the information will now be described. The display 408 simultaneously display two perspective images (two-direction perspective images) output as second images from the second image acquirer 402 next to each other in a pre-established region. The display 408 displays information of the closed space output from the learner 404 overlaid onto the corresponding perspective images. When this is done, if the information of the closed space output from the learner 404 is, for example, information of a three-dimensional space, the display 408, by using a projection matrix determined from the geometry of each of the ray detector 13-1 and the ray detector 13-2 of the treatment apparatus 10, displays an image representing the range of the closed space overlaid onto the corresponding perspective images. Additionally, the display 408 displays information of the position of the characteristic subject represented by the second characteristic output from the second characteristic calculator 4032 overlaid onto the corresponding perspective images. When this is done, the display 408 displays a visually easy-to-grasp symbol or icon at the position of the characteristic subject represented by the second characteristic, overlaid onto the corresponding perspective images. In this case, if the second characteristics are output from the second characteristic calculator 4032 in time sequence, by updating the positions of the characteristic subject represented by each of the second characteristics, the manner in which the characteristic subject moves can be presented. In this case, if the positions of the characteristic subject represented by the second characteristics are not updated, but rather are added to the display, the trace of the movement of the characteristic subject can be presented.

In the learner 404 outputs information of the gate window, the display 408 displays an image representing the range of the gate window overlaid onto the corresponding perspective images. If the determiner 405 outputs information of the distance between the position of the characteristic subject represented by the second characteristic and the boundary of the range of movement of the characteristic subject represented by the closed space, the display 408 displays a numerical stream representing information of the distance in the periphery of the corresponding perspective image, outside the region in which the perspective images are displayed next to each other. When this is done, the display 408, based on the value (size) of the distance, may change the color of the numerical stream representing the information of the displayed distance, in accordance with a color chart.

The display 408 displays overlaid information indicated by the determination signal output from the determiner 405. For example, if the information indicated by the determination signal output from the determiner 405 indicates that an abnormality has been detected, the display 408 displays overlaid largely onto the entire screen a message notifying that an abnormality has occurred. The message notifying that an abnormality has occurred may be overlaid onto the current display as a so-called pop-up screen. This enables a warning to be issued to the user (a physician or the like) of the treatment system 4 that has the medical image processing apparatus 400.

The constitution with which a user (a physician or the like) of the treatment system 4 that has the medical image processing apparatus 400 is issued a warning is not restricted to the display 408 shown in FIG. 7. For example, the display 408 constitution may have a speech output that generates an alarm sound if a message or pop-up screen notifying that an abnormality has occurred is displayed. The alarm to the user (a physician or the like) can be thought to be sufficient if it can at least notify that an abnormality has occurred, in which case a speech output may be provided in place of the display 408 of the medical image processing apparatus 400, and speech output may notify with only an alarm sound that an abnormality has occurred.

By this constitution and operation, the medical image processing apparatus 400, similar to the medical image processing apparatus 100 of the first embodiment, based on a first image of the patient P captured before treatment and a second image of the patient P captured during treatment, outputs as a determination signal the results of determining the offset between a gate window established in the treatment plan and the current position of the characteristic subject in the body of the patient P. In the treatment system 4 that has the medical image processing apparatus 400, similar to the treatment system 1 that has the medical image processing apparatus 100 of the first embodiment, this enables irradiation by the treatment beam B at an appropriate timing that is synchronized to the exhalation and inhalation by the patient P and development of a strategy for the case in which an unexpected situation occurs in the patient P during treatment.

Furthermore, in the medical image processing apparatus 400, the display 408 displays information output from the constituent elements of the medical image processing apparatus 400 as images. In the medical image processing apparatus 400, this enables visual recognition of the state during treatment. By doing this, in the treatment system 4 that has the medical image processing apparatus 400, it is possible to perform safe radiotherapy while visually verifying the status of tracking the characteristic subject in the body of the patient P during treatment.

The state detection processing in the medical image processing apparatus 400, except for the display 408 displaying information at each processing stage, is the same as the state detection processing in the medical image processing apparatus 100 of the first embodiment shown in FIG. 3. Therefore, the detailed description regarding the flow of the state detection processing in the medical image processing apparatus 400 will be omitted.

As described above, in the medical image processing apparatus 400 of the fourth embodiment, similar to the medical image processing apparatus 100 of the first embodiment, the position of the characteristic subject within the body of the patient P during treatment is tracked, and a determination signal representing the tracking results is output. In the treatment system 4 having the medical image processing apparatus 400 of the fourth embodiment, similar to the treatment system 1 having the medical image processing apparatus 100 of the first embodiment, this enables irradiation by the treatment beam B with appropriate timing synchronized to the exhalation and inhalation by the patient P and the development of a strategy in the case of an unexpected situation occurring in the patient P during treatment being detected.

In the medical image processing apparatus 400 of the fourth embodiment, the display 408 displays the state of tracking of the position of the characteristic subject within the body of the patient P during treatment. In the treatment system 4 having the medical image processing apparatus 400 of the fourth embodiment, this enables safe radiotherapy while verifying the current position of the characteristic subject. In the medical image processing apparatus 400 of the fourth embodiment, if an unexpected situation occurring in the patient P during treatment is detected, the display 408 displays an alarm. In the treatment system 4 having the medical image processing apparatus 400 of the fourth embodiment, this enables the development of an appropriate strategy to handle an unexpected situation occurring in the patient P during treatment. The constitution need not be one in which the display 408 is provided in the medical image processing apparatus 400 of the fourth embodiment, and may be one in which it is provided in the treatment system 4 that has the medical image processing apparatus 400 of the fourth embodiment.

In the medical image processing apparatus 400 of the fourth embodiment, in the constitution of the medical image processing apparatus 400 shown in FIG. 7, the description has been for the case of a constitution in which the display 408 generates and displays (overlaid) an image in accordance with information output from constituent elements of the medical image processing apparatus 400. However, the image displayed (overlaid) by the display 408 is not restricted to a constitution in which it is generated by the display 408, and the constitution may be one in which the image is generated by each of the constituent elements that output the information. For example, the constitution may be one in which, in the medical image processing apparatus 400, the second image acquirer 402 generates one image for simultaneous display of two perspective images (two-direction perspective images) next to each other on a pre-established region, and the generated one image is output to the display 408 as the second image. In this case, the constitution is such that the display 408 displays the one image output from the second image acquirer 402 as is. Also, for example, in the medical image processing apparatus 400, constitution may be such that the learner 404 generates an image representing the range of the closed space for overlaying onto the perspective image, and outputs the generated image to the display 408 as closed space information. When this is done, the learner 404 may output to the display 408, as closed space information, one image that includes each of the images representing the range of the closed space to be displayed overlaid onto each of the perspective images next to each other in one image generated by the second image acquirer 402. In this case, the constitution may be one in which the display 408 overlays one image output from the learner 404 as is onto one image output from the second image acquirer 402.

As described above, the medical image processing apparatus 400 further has a display 408 that displays the results of the determination by the determiner 405.

Also, as described above, in the medical image processing apparatus 400, the display 408 may display the second image and displays the range of the closed space overlaid onto the display of the second image.

(Fifth Embodiment)

The fifth embodiment will now be described. The constitution of a treatment system having a medical image processing apparatus of the fifth embodiment is the constitution of the treatment system 1 having the medical image processing apparatus 100 of the first embodiment as shown in FIG. 1, with the medical image processing apparatus 100 replaced by the medical image processing apparatus of the fifth embodiment (hereinafter, the "medical image processing apparatus 500"). In the description to follow, the treatment system having the medical image processing apparatus 500 will be referred to as the treatment system 5.

In the description to follow, of the constituent elements of the treatment system 5 having the medical image processing apparatus 500, constituent elements that are the same as constituent elements of the treatment system 1 having the medical image processing apparatus 100 of the first embodiment are assigned the same reference symbols and the detailed descriptions of those constituent elements will be omitted. In the following, only the constitution, operation, and processing of the medical image processing apparatus 500, which is a constituent element different from the medical image processing apparatus 100 of the first embodiment, will be described.

The medical image processing apparatus 500, similar to the medical image processing apparatus 100 of the first embodiment, tracks the characteristic subject within the body of the patient P to be treated by radiotherapy, automatically detects the timing of the irradiation by the treatment beam B and an unexpected situation, and outputs the detected information. The medical image processing apparatus 500, based on the results of tracking the characteristic subject within the body of the patient P, controls the irradiation with the treatment beam B by the treatment beam irradiation gantry 14 of the treatment apparatus 10 and the irradiation with the radio beam r by the ray irradiator 12.

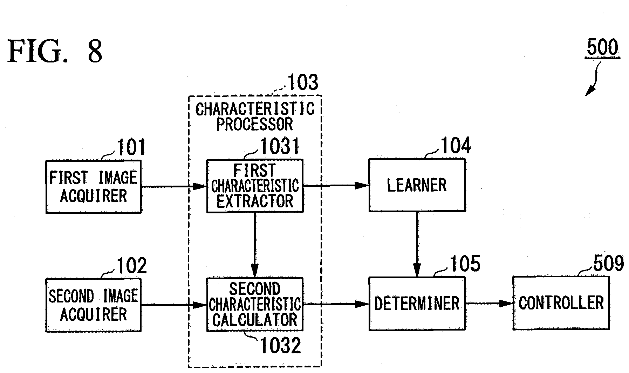

The constitution of the medical image processing apparatus 500 of treatment system 5 will now be described. FIG. 8 is a block diagram showing the general constitution of the medical image processing apparatus 500 of the fifth embodiment. The medical image processing apparatus 500 shown in FIG. 8 has a first image acquirer 101, a second image acquirer 102, a characteristic processor 103, a learner 104, a determiner 105, and a controller 509. The characteristic processor 103 has a first characteristic extractor 1031 and a second characteristic calculator 1032.

The medical image processing apparatus 500 has the constitution of the medical image processing apparatus 100 of the first embodiment, to which the controller 509 is added. The other constituent elements of the medical image processing apparatus 500 are the same as the constituent elements of the medical image processing apparatus 100 of the first embodiment. Therefore, in the description to follow, of the constituent elements of the medical image processing apparatus 500, ones that are the same as constituent elements in the medical image processing apparatus 100 of the first embodiment are assigned the same reference numerals, and the detailed descriptions thereof will be omitted. In the description to follow, only constituent elements different from the medical image processing apparatus 100 of the first embodiment will be described.

The determiner 105, based on information of a closed space output from the learner 104 and a second characteristic output from the second characteristic calculator 1032 of the characteristic processor 103, outputs to the controller 509 a determination signal indicating the result of determining whether or not the second characteristic is within the closed space.

The controller 509, based on the determination signal output from the determiner 105, controls the radiotherapy in the treatment system 5 that has the medical image processing apparatus 500. That is, the controller 509 performs control so as to perform radiotherapy in the treatment system 5 if the determination signal indicates that no abnormality has been detected, and so as not to perform radiotherapy in the treatment system 5 if the determination signal indicates that an abnormality has been detected.

In the control of the radiotherapy by the controller 509, for example, the irradiation with the treatment beam B by the treatment beam irradiation gantry 14 of the treatment apparatus 10 and the capturing of a perspective image by the ray irradiator 12 and the ray detector 13 are controlled. More specifically, if the determination signal indicates that an abnormality has not been detected and that the current characteristic subject position is within the range of the closed space, the controller 509 controls so as to cause the ray irradiator 12 to irradiate with the radio beam r and the ray detector 13 to detect the radio beam r that has reached it by passing through the patient P and to generate a perspective, image of the inside of the body of the patient P. If the determination signal indicates that an abnormality has not been detected and that the current position of the characteristic subject has come to within the gate window established in the treatment plan, the controller 509 controls so as to cause the treatment beam irradiation gantry 14 to irradiate with the treatment beam B.

If the determination signal indicates that an abnormality has been detected, the controller 509 performs control so as to stop the irradiation by the treatment beam irradiation gantry 14 with the treatment beam B. When this occurs, the controller 509 may perform control so as to stop the irradiation with the radio beam r by the ray irradiator 12 and the generation of perspective image by the ray detector 13, that is, so as to stop the capturing of the perspective image (second image). Performing this control enables avoidance of unnecessary irradiation of the patient P by the radio beam r.

Unexpected situations that can be envisioned as factors in the determination signal indicating the detection of an abnormality are unexpected situation such as coughing and sneezing by the patient P and the occurrence of the apnea syndrome when the patient P is sleeping, which do not require a long time to be corrected. That is, these are situations in which, for example, it is envisioned that the coughing or sneezing by the patient P will subside, enabling radiotherapy in a stable state. For that reason, if the determination signal indicates that an abnormality has been detected, rather than immediately stopping the capture of the perspective image (second image), the controller 509 may perform control so as to lengthen the interval of capturing the perspective image for a pre-established length of time, after which if the determination signal still indicates that an abnormality has been detected, the capture of the perspective image is stopped.

By this constitution and operation, the medical image processing apparatus 500, similar to the medical image processing apparatus 100 of the first embodiment, based on the first image of the patient P captured before treatment and the second image of the patient P captured during treatment, automatically detects the timing of irradiation by the treatment beam B and unexpected situations. In the medical image processing apparatus 500, based on the detection result, the controller 509 controls the irradiation with the treatment beam B by the treatment, beam irradiation gantry 14 of the treatment apparatus 10 and the irradiation with the radio beam r by the ray irradiator 12. In particular, if the determination signal indicates that an abnormality has been detected, the controller 509 performs controls so as to stop the irradiation of the patient P with the treatment beam B and the radio beam r. In the treatment system 5 having the medical image processing apparatus 500, this enables radiotherapy to be performed safely.

The state detection processing in the medical image processing apparatus 500 can be easily thought of as being the state detection processing in the medical image processing apparatus 100 of the first embodiment shown in FIG. 3, with the processing by the controller 509 added thereto. More specifically, this can be easily envisioned by, in the state detection processing of the medical image processing apparatus 100 of the first embodiment shown in FIG. 3, adding the processing to control by the controller 509 the irradiation with the treatment beam B and the radio beam r before returning to step S400 after the processing of step S600. Therefore, the detailed description of the flow of the state detection processing in the medical image processing apparatus 500 will be omitted.

As described above, in the medical image processing apparatus 500 of the fifth embodiment, similar to the medical image processing apparatus 100 of the first embodiment, the determination signal is output that indicates the result of tracking and determining the position of the characteristic subject within the body of the patient P. In the medical image processing apparatus 500 of the fifth embodiment, the irradiation of the treatment beam B and the radio beam r in the treatment apparatus 10 is controlled based on the determination signal. In the treatment system 5 that has the medical image processing apparatus 500 of the fifth embodiment, this enables safe radiotherapy, in accordance with the state of tracking the position of the characteristic subject within the body of the patient P during treatment.

In the medical image processing apparatus 500 of the fifth embodiment, the description has been for the case in which the controller 509 is provided as a constituent element of the medical image processing apparatus 500 in the constitution of the medical image processing apparatus 500 as shown in FIG. 8. However, the controller 509 is not restricted to being a constituent element of the medical image processing apparatus 500. For example, in a treatment system 5 that has the medical image processing apparatus 500 of the fifth embodiment, the function of the controller 509, that is, that function of controlling the irradiation with the treatment beam B and the radio beam r based on the determiner output from the determiner 105 of the medical image processing apparatus 500, may be provided in the treatment apparatus 10.

As described above, in the medical image processing apparatus 500, the determination signal is transmitted to the controller that controls the treatment apparatus 10.

As described above, in the medical image processing apparatuses of the embodiments, based on the first image of the patient P captured before treatment and the second image of the patient P captured during treatment, the position of the characteristic subject within the body of the patient P is tracked during treatment, and a determination signal is output that indicates the timing of irradiation with the treatment beam B of a lesion in the body of the patient P during treatment by irradiation and the result of detecting an unexpected situation occurring in the patient P during treatment is output. In a treatment system that has a medical image processing apparatus of the embodiments, this enables development of a strategy in the event that an unexpected situation is detected in the patient P during treatment and enables safe treatment by irradiation with the treatment beam B with an appropriate timing, synchronized to the exhalation and inhalation by the patient P.

In the second embodiment to the fifth embodiment, the descriptions have been for the addition of constituent elements that are features of each of the embodiments to the medical image processing apparatus 100 of the first embodiment. However, there is no restriction to a constitution in which the constituent elements that are features of each of the embodiments are each exclusively provided in the medical image processing apparatus. That is, the constituent elements that are the features of each of the embodiments may be provided simultaneously in the medical image processing apparatus. For example, a medical image processing apparatus may be constituted to have simultaneously have the display 408 provided in the medical image processing apparatus 400 of the fourth embodiment and the controller 509 provided in the medical image processing apparatus 500 of the fifth embodiment. In this case, the other constituent elements provided in the medical image processing apparatus can be appropriately changed to implement the functions corresponding to each of those constituent elements.

In each of the embodiments, the description has been for constitutions in which the medical image processing apparatus and the treatment apparatus 10 are separated. However, the constitution is not restricted to one in which the medical image processing apparatus and the treatment apparatus 10 are separated, and the medical image processing apparatus and the treatment apparatus 10 may be constituted as one.

A medical image processing program used in a treatment system described in the above-noted embodiments is a medical image processing program for causing a computer to function as a medical image processing apparatus that has a first image acquirer that acquires a plurality of first images of an object to be treated captured at a plurality of times by a radiographic imaging apparatus, a second image acquirer that acquires a second image of the object to be treated captured by a radiographic imaging apparatus at a time different from the first images, a characteristic processor that determines a plurality of positions of a first characteristic of the object to be treated from the plurality of first images and that determines the position of a second characteristic corresponding to the first characteristic in the object to be treated from the second image, a calculator that establishes a first closed space that includes a plurality of positions of the first characteristic, and a determiner that outputs a determination signal, based on the result of determining whether or not the position of the second characteristic is within the first closed space.

According to at least one of the above-described embodiments, by having a first image acquirer (101) that acquires a plurality of first images of an object to be treated (patient P) captured by a radiographic imaging apparatus at a plurality of times, a second image acquirer (102) that acquires a second image of the patient P captured by a radiographic imaging apparatus (two sets of a ray irradiator 12 and a ray detector 13) at a time different from the first images, a characteristic processor (103) that determines a plurality of positions of a first characteristic of the patient P from the plurality of first images and that determines the position of a second characteristic corresponding to the first characteristic in the body of the patient P from the second image, a calculation unit (a learner 104) that establishes a first closed space (closed space) that includes the plurality of positions of the first characteristic, and a determiner (105) that outputs a determination signal based on the result of determining whether or not the position of the second characteristic is within the first closed space, it is possible to automatically detect an unexpected situation occurring during irradiation by a radio beam in radiotherapy.

A program for implementing the functions of each of the constituent elements of a medical image processing apparatus, such as the characteristic processor 103 that includes the first characteristic extractor 1031 and the second characteristic calculator 1032, the learner 104, the determiner 105, and the like, may be recorded in a computer-readable storage medium, and a computer system may be made to read-in and execute the program stored in the storage medium, so as to implement the various above-noted functions of treatment system of the present embodiment. The term "computer system" may include an operating system and hardware such as peripheral devices. The term "computer system" also includes a WWW system having a webpage-providing environment (or webpage-displaying environment). The term "computer-readable storage medium" refers to a writable non-volatile memory such as a flexible disk, an optomagnetic disk, a ROM, a flash memory, a removable media such as a CD-ROM, or the like, or a storage device such as a hard disk or the like built into a computer system.

Additionally, the term "computer-readable storage medium" encompasses one holding a program for a given period of time, such as a volatile memory (DRAM: dynamic random access memory) within a computer system serving as a server or client when a program is transmitted via a network such as the Internet or via a communication line such as a telephone line. The above-noted program may be transferred from a computer system in which the program is stored in a storage apparatus to another computer system, either via a transfer medium, or by a transfer wave in a transfer medium. In this case, the term "transfer medium" transferring a program refers to a medium having a function of transferring information, such as a network (communication network) such as the Internet, or a communication circuit (communication line) such as a telephone line. The above-noted program-may be for implementing a part of the above-described functionality. Additionally, it may be a so-called difference file (difference program) enabling implementation in combination with a program that already has recorded the above-noted functionality in a computer system.

Each element for the medical image processing apparatus described above and for the treatment system described above can be implemented by hardware with or without software. In some cases, the medical image processing apparatus may be implemented by one or more hardware processors and one or more software components wherein the one or more software components are to be executed by the one or more hardware processors to implement each element for the medical image processing apparatus and for the treatment system. In some other cases, the medical image processing apparatus may be implemented by a system of circuits or circuitry configured to perform each operation of each element for the medical image processing apparatus and for the treatment system.

While certain embodiments of the present inventions have been described, these embodiments have been presented by way of example only, and are not intended to limit the scope of the inventions.

**Claims**

1. A medical image processing apparatus (100, 200, 300, 400) comprising:

   a first image acquirer (101) that acquires a plurality of first images of an object to be treated, each of the plurality of first images being captured by a radiographic imaging apparatus at a respective time of a plurality of times;
   a second image acquirer (102) that acquires a second image of the object to be treated ,the second image being captured by a radiographic imaging apparatus at a time different from the plurality of times when the plurality of first images are captured;
   a characteristic processor (103) that determines a plurality of positions of a first characteristic of the object to be treated from the plurality of first images, the characteristic processor that determines the position of a second characteristic corresponding to the first characteristic in the object to be treated from the second image;
   a calculator (104) that establishes a first closed space including the plurality of positions of the first characteristic; and
   a determiner (105) that determines whether or not the position of the second characteristic is within the first closed space, and outputs a determination signal, based on a result of the determination.

2. The medical image processing apparatus according to claim 1, wherein the characteristic processor comprises:

   a first characteristic extractor (1031) that calculates a characteristic extraction parameter for extracting a characteristic subject and that outputs information representing the position of the characteristic subject extracted in accordance with the characteristic extraction parameter as the first characteristic; and
   a second characteristic calculator (1032) that extracts the characteristic subject based on the characteristic extraction parameter and that outputs, as the second characteristic, information representing the position of the characteristic subject corresponding to the first characteristic.

3. The medical image processing apparatus according to claim 2,
   wherein the determiner outputs the determination signal based on the results of determining whether or not the second characteristic is within a second closed space included in the first closed space.

4. The medical image processing apparatus according to claim 3, further comprising:
   a classifier (206) that classifies the plurality of first characteristic positions into two or more classes, thereby setting the second closed space from the first closed space.

5. The medical image processing apparatus according to any one of claims 2 to 4,
   wherein the first characteristic extraction unit calculates the plurality of characteristic extraction parameters and outputs the plurality of first characteristics corresponding to each of the characteristic extraction parameters; and
   the apparatus further has a selector (307) that, based on the distribution of the position of the characteristic subject represented by each of the plurality of first characteristics, selects the characteristic extraction parameter used for extraction of the characteristic subject by the second characteristic calculator and the first characteristic used for establishing the first closed space by the calculator.

6. The medical image processing apparatus according to claim 5, wherein the apparatus is adapted for displaying the second image and displaying the range of the first closed space overlaid onto the display of the second image.

7. The medical image processing apparatus according to any one of claims 2 to 6, wherein the determination signal is transmitted to a controller that controls a treatment apparatus.

8. The medical image processing apparatus according to any one of claims 2 to 7, wherein the first characteristic is a marker in the object to be treated.

9. The medical image processing apparatus according to claim 5, wherein the characteristic extraction parameter is an image of a region of interest for specifying a partial image region that includes the characteristic subject within the object to be treated captured in the first and second images.

10. A treatment system (1,2,3,4,5) comprising:

    a medical image processing apparatus according to any one of claims 1 to 9;

a treatment apparatus that has a ray irradiator irradiating the subject to be treated with the treatment beam and the radiographic imaging apparatuses capturing the first and second images; and

a controller that controls the irradiation of the treatment beam by the ray irradiator, based on the determination signal.

**11.** The treatment system according to claim 10, further comprising:
a display that displays the result of determination by the determiner.

**12.** A non-transitory computer readable storage medium that stores a computer program which causes, when executed by a computing device, a medical image processing program for causing a computer to function as a medical image processing apparatus to perform at least:

acquiring a plurality of first images of an object to be treated captured at a plurality of times by a radiographic imaging apparatus;

acquiring a second image of the object to be treated captured by a radiographic imaging apparatus at a time different from the first images;

determining a plurality of positions of a first characteristic of the object to be treated from the plurality of first images and that determines the position of a second characteristic corresponding to the first characteristic in the object to be treated from the second image;

establishing a first closed space that includes a plurality of positions of the first characteristic; and

determining whether or not the position of the second characteristic is within the first closed space, and outputting a determination signal, based on a result of the determination.

**Patentansprüche**

**1.** Medizinisches Bildverarbeitungsgerät (100, 200, 300, 400), das Folgendes umfasst:

einen ersten Bildaufzeichner (101), der eine Vielzahl erster Bilder eines Objekts, das zu behandeln ist, aufzeichnet, wobei jedes der Vielzahl erster Bilder von einem Röntgenbildgebungsgerät an einem jeweiligen Zeitpunkt einer Vielzahl von Zeitpunkten erfasst wird;

einen zweiten Bildaufzeichner (102), der ein zweites Bild des Objekts, das zu behandeln ist, aufnimmt, wobei das zweite Bild von einem Röntgenbildgebungsgerät an einem Zeitpunkt erfasst wird, der von der Vielzahl von Zeitpunkten, wenn die Vielzahl erster Bilder erfasst wird, unterschiedlich ist;

einen Merkmalprozessor (103), der eine Vielzahl von Positionen eines ersten Merkmals des Objekts, das zu behandeln ist, aus einer Vielzahl erster Bilder bestimmt, wobei der Merkmalprozessor die Position eines zweiten Merkmals, das dem ersten Merkmal in dem Objekt, das zu behandeln ist, entspricht, aus dem zweiten Bild bestimmt;

einen Rechner (104), der einen ersten geschlossenen Raum festlegt, der die Vielzahl von Positionen des ersten Merkmals beinhaltet; und

einen Bestimmer (105), der bestimmt, ob die Position des zweiten Merkmals innerhalb des ersten geschlossenen Raums liegt, und ein Bestimmungssignal, das auf einem Resultat der Bestimmung basiert, ausgibt.

**2.** Medizinisches Bildverarbeitungsgerät nach Anspruch 1, wobei der Merkmalprozessor Folgendes umfasst:

einen ersten Merkmalextraktor (1031), der einen Merkmalextraktionsparameter für das Extrahieren eines Merkmalsubjekts berechnet und Informationen ausgibt, die die Position des Merkmalsubjekts darstellen, das in Übereinstimmung mit dem Merkmalextraktionsparameter als das erste Merkmal extrahiert wird; und

einen zweiten Merkmalberechner (1032), der das Merkmalsubjekt basierend auf dem Merkmalextraktionsparameter extrahiert und der als das zweite Merkmal Informationen ausgibt, die die Position des Merkmalsubjekts, das dem ersten Merkmal entspricht, darstellen.

**3.** Medizinisches Bildverarbeitungsgerät nach Anspruch 2,
wobei der Bestimmer das Bestimmungssignal basierend auf den Resultaten des Bestimmens, ob das zweite Merkmal innerhalb eines zweiten geschlossenen Raums, der in dem ersten geschlossenen Raum enthalten ist, liegt oder nicht.

**4.** Medizinisches Bildverarbeitungsgerät nach Anspruch 3, das weiter Folgendes umfasst:
einen Klassierer (206), der die Vielzahl erster Merkmalpositionen in zwei oder mehr Klassen klassiert, wodurch der zweite geschlossene Raum von dem ersten geschlossenen Raum gestellt wird.

5. Medizinisches Bildverarbeitungsgerät nach einem der Ansprüche 2 bis 4, wobei die erste Merkmalextraktionseinheit die Vielzahl von Merkmalextraktionsparametern berechnet und die Vielzahl erster Merkmale ausgibt, die jedem der Merkmalextraktionsparameter entspricht; und das Gerät weiter einen Auswähler (307) aufweist, der, basierend auf der Verteilung der Position des Merkmalsubjekts, die von jedem der Vielzahl erster Merkmale dargestellt ist, den Merkmalextraktionsparameter auswählt, der für das Extrahieren des Merkmalsubjekts durch den zweiten Merkmalberechner verwendet wird, und das erste Merkmal, das zum Festlegen des ersten geschlossenen Raums durch den Rechner verwendet wird.

6. Medizinisches Bildverarbeitungsgerät nach Anspruch 5, wobei das Gerät angepasst ist, um das zweite Bild anzuzeigen und den Bereich des ersten geschlossenen Raums auf dem Display des zweiten Bilds überlagert anzuzeigen.

7. Medizinisches Bildverarbeitungsgerät nach einem der Ansprüche 2 bis 6, wobei das Bestimmungssignal zu einer Steuervorrichtung, die ein Behandlungsgerät steuert, übertragen wird.

8. Medizinisches Bildverarbeitungsgerät nach einem der Ansprüche 2 bis 7, wobei das erste Merkmal ein Marker in dem Objekt, das zu behandeln ist, ist.

9. Medizinisches Bildverarbeitungsgerät nach Anspruch 5, wobei der Merkmalextraktionsparameter ein Bild eines Interessensbereichs zum Spezifizieren eines teilweisen Bildbereichs ist, der das Merkmalsubjekt innerhalb des Objekts, das zu behandeln ist, das in dem ersten und zweiten Bild erfasst ist, beinhaltet.

10. Behandlungssystem (1, 2, 3, 4, 5), das Folgendes umfasst:

ein medizinisches Bildverarbeitungsgerät nach einem der Ansprüche 1 bis 9;
ein Behandlungsgerät, das einen Bestrahler, der das zu behandelnde Subjekt mit dem Behandlungsstrahl bestrahlt, und die Röntgenbildgebungsgeräte, die das erste und das zweite Bild erfassen, aufweist; und
eine Steuervorrichtung, die die Bestrahlung des Behandlungsstrahls durch den Bestrahler basierend auf dem Bestimmungssignal steuert.

11. Behandlungssystem nach Anspruch 10, das weiter Folgendes umfasst:

ein Display, das das Resultat der Bestimmung durch den Bestimmer anzeigt.

12. Nichtflüchtiges computerlesbares Speichermedium, das ein Computerprogramm speichert, das, wenn es von einem Rechengerät ausgeführt wird, ein medizinisches Bildverarbeitungsprogramm veranlasst, einen Computer zu veranlassen, als ein medizinisches Bildverarbeitungsgerät zu funktionieren, um mindestens Folgendes auszuführen:

Aufzeichnen einer Vielzahl erster Bilder eines Objekts, das zu behandeln ist, die an einer Vielzahl von Zeitpunkten von einem Röntgenbildgebungsgerät erfasst werden;
Aufzeichnen eines zweiten Bilds des Objekts, das zu behandeln ist, das von einem Röntgenbildgebungsgerät an einem Zeitpunkt, der von den ersten Bildern unterschiedlich ist, erfasst wird;
Bestimmen einer Vielzahl von Positionen eines ersten Merkmals des Objekts, das zu behandeln ist, aus der Vielzahl erster Bilder, und das die Position eines zweiten Merkmals bestimmt, das dem ersten Merkmal in dem Objekt, das zu behandeln ist, entspricht, aus dem zweiten Bild;
Festlegen eines ersten geschlossenen Raums, der eine Vielzahl von Positionen des ersten Merkmals beinhaltet; und
Bestimmen, ob die Position des zweiten Merkmals innerhalb des ersten Raums liegt oder nicht, und Ausgeben eines Bestimmungssignals basierend auf einem Resultat der Bestimmung.

**Revendications**

1. Appareil de traitement d'images médicales (100, 200, 300, 400) comprenant :

un premier dispositif d'acquisition d'images (101) qui acquiert une pluralité de premières images d'un objet devant être traité, chacune de la pluralité de premières images étant capturée par un appareil d'imagerie radiographique à un instant respectif d'une pluralité d'instants ;

un second dispositif d'acquisition d'image (102) qui acquiert une seconde image de l'objet devant être traité, la seconde image étant capturée par un appareil d'imagerie radiographique à un instant différent de la pluralité d'instants lorsque la pluralité de premières images sont capturées ;

un processeur de caractéristiques (103) qui détermine une pluralité de positions d'une première caractéristique de l'objet devant être traité à partir de la pluralité de premières images, le processeur de caractéristiques qui détermine la position d'une seconde caractéristique correspondant à la première caractéristique dans l'objet devant être traité à partir de la seconde image ;

un calculateur (104) qui établit un premier espace fermé incluant la pluralité de positions de la première caractéristique ; et

un dispositif de détermination (105) qui détermine si la position de la seconde caractéristique est ou non à l'intérieur du premier espace fermé, et délivre en sortie un signal de détermination, sur la base d'un résultat de la détermination.

2. Appareil de traitement d'images médicales selon la revendication 1, dans lequel le processeur de caractéristiques comprend :

un premier extracteur de caractéristique (1031) qui calcule un paramètre d'extraction de caractéristique pour extraire un sujet caractéristique et qui délivre en sortie des informations représentant la position du sujet caractéristique extrait conformément au paramètre d'extraction de caractéristique en tant que première caractéristique ; et

un second calculateur de caractéristique (1032) qui extrait le sujet caractéristique sur la base du paramètre d'extraction de caractéristique et qui délivre en sortie, en tant que seconde caractéristique, des informations représentant la position du sujet caractéristique correspondant à la première caractéristique.

3. Appareil de traitement d'images médicales selon la revendication 2,
dans lequel le dispositif de détermination délivre en sortie le signal de détermination sur la base des résultats de la détermination que la seconde caractéristique est ou non à l'intérieur d'un second espace fermé inclus dans le premier espace fermé.

4. Appareil de traitement d'images médicales selon la revendication 3, comprenant en outre :
un dispositif de classification (206) qui classe la pluralité de positions de la première caractéristique en deux classes ou plus, en définissant ainsi le second espace fermé à partir du premier espace fermé.

5. Appareil de traitement d'images médicales selon l'une quelconque des revendications 2 à 4,
dans lequel la première unité d'extraction de caractéristique calcule la pluralité de paramètres d'extraction de caractéristique et délivre en sortie la pluralité de premières caractéristiques correspondant à chacun des paramètres d'extraction de caractéristique ; et
l'appareil comporte en outre un sélecteur (307) qui, sur la base de la distribution de la position du sujet caractéristique représenté par chacune de la pluralité de premières caractéristiques, sélectionne le paramètre d'extraction de caractéristique utilisé pour une extraction du sujet caractéristique par le second calculateur de caractéristique et de la première caractéristique utilisée pour établir le premier espace fermé par le calculateur.

6. Appareil de traitement d'images médicales selon la revendication 5, dans lequel l'appareil est adapté pour afficher la seconde image et afficher l'étendue du premier espace fermé chevauchant l'affichage de la seconde image.

7. Appareil de traitement d'images médicales selon l'une quelconque des revendications 2 à 6, dans lequel le signal de détermination est transmis à un dispositif de commande qui commande un appareil de traitement.

8. Appareil de traitement d'images médicales selon l'une quelconque des revendications 2 à 7, dans lequel la première caractéristique est un marqueur dans l'objet devant être traité.

9. Appareil de traitement d'images médicales selon la revendication 5, dans lequel le paramètre d'extraction de caractéristique est une image d'une région d'intérêt pour spécifier une région d'image partielle qui inclut le sujet caractéristique à l'intérieur de l'objet devant être traité capturée dans les premières et seconde images.

10. Système de traitement (1, 2, 3, 4, 5) comprenant :

un appareil de traitement d'images médicales selon l'une quelconque des revendications 1 à 9 ;
un appareil de traitement qui comporte un irradiateur de rayonnement irradiant le sujet devant être traité par le faisceau de traitement et les appareils d'imagerie radiographique capturant les premières et seconde images ; et
un dispositif de commande qui commande l'irradiation du faisceau de traitement par l'irradiateur de rayonnement, sur la base du signal de détermination.

11. Système de traitement selon la revendication 10, comprenant en outre :
une unité d'affichage qui affiche le résultat de détermination par le dispositif de détermination.

12. Support de stockage lisible par ordinateur non transitoire qui stocke un programme informatique qui amène, lorsqu'il est exécuté par un dispositif informatique, un programme de traitement d'images médicales à amener un ordinateur à fonctionner en tant qu'appareil de traitement d'images médicales à réaliser au moins les étapes :

d'acquisition d'une pluralité de premières images d'un objet devant être traité capturées à une pluralité d'instants par un appareil d'imagerie radiographique ;
d'acquisition d'une seconde image de l'objet devant être traité capturée par un appareil d'imagerie radiographique à un instant différent des premières images ;
de détermination d'une pluralité de positions d'une première caractéristique de l'objet devant être traité à partir de la pluralité de premières images et qui détermine la position d'une seconde caractéristique correspondant à la première caractéristique dans l'objet devant être traité à partir de la seconde image ;
d'établissement d'un premier espace fermé qui inclut une pluralité de positions de la première caractéristique ; et
de détermination que la position de la seconde caractéristique est ou non à l'intérieur du premier espace fermé, et de délivrance en sortie d'un signal de détermination, sur la base d'un résultat de la détermination.

## FIG. 1

## FIG. 2

# FIG. 3

```
           ┌──────────────┐
           │    START     │
           └──────┬───────┘
                  │           ╱S100
         ┌────────▼─────────┐
         │ ACQUIRE FIRST IMAGE │
         └────────┬─────────┘
                  │           ╱S200
         ┌────────▼─────────┐
         │ EXTRACT FIRST CHARACTERISTIC │
         │   FROM THE FIRST IMAGE       │
         └────────┬─────────┘
                  │           ╱S300
         ┌────────▼──────────────────────┐
         │ ESTABLISH CLOSED SPACE BASED ON │
         │ CHARACTERISTIC SUBJECT POSITION │
         │ DISTRIBUTION AT A PLURALITY OF TIMES │
         │ REPRESENTED BY FIRST CHARACTERISTIC │
         └────────┬──────────────────────┘
                  │           ╱S400
         ┌────────▼─────────┐
         │ ACQUIRE SECOND IMAGE │
         └────────┬─────────┘
                  │           ╱S500
         ┌────────▼─────────┐
         │ CALCULATE SECOND CHARACTERISTIC │
         │    FROM SECOND IMAGE           │
         └────────┬─────────┘
                  │           ╱S600
         ┌────────▼──────────────────────┐
         │ DETERMINE WHETHER THE SECOND    │
         │ CHARACTERISTIC IS WITHIN RANGE OF CLOSED │
         │ SPACE AND OUTPUT DETERMINATION SIGNAL │
         └────────┬──────────────────────┘
                  │
```

# FIG. 4

(a)                              (b)

# FIG. 5

<u>200</u>

CHARACTERISTIC PROCESSOR 103

101 FIRST IMAGE ACQUIRER

1031 FIRST CHARACTERISTIC EXTRACTOR

206 CLASSIFIER

204 LEARNER

102 SECOND IMAGE ACQUIRER

1032 SECOND CHARACTERISTIC CALCULATOR

205 DETERMINER

DETERMINATION SIGNAL

FIG. 6

_300_

CHARACTERISTIC PROCESSOR _303_

| 101 FIRST IMAGE ACQUIRER | → | 3031 FIRST CHARACTERISTIC EXTRACTOR | → | 307 SELECTOR | → | 104 LEARNER |

| 102 SECOND IMAGE ACQUIRER | → | SECOND CHARACTERISTIC CALCULATOR 3032 | → | 105 DETERMINER | → DETERMINATION SIGNAL |

FIG. 7

_400_

CHARACTERISTIC PROCESSOR _403_

| 101 FIRST IMAGE ACQUIRER | → | 1031 FIRST CHARACTERISTIC EXTRACTOR | → | 404 LEARNER | → | 408 DISPLAY |

| 402 SECOND IMAGE ACQUIRER | → | SECOND CHARACTERISTIC CALCULATOR 4032 | → | 405 DETERMINER | → DETERMINATION SIGNAL |

32

FIG. 8                                                                500

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5925233 B

- US 2016148401 A1